# EUROPEAN PATENT SPECIFICATION

(11) **EP 4 341 236 B1**
(45) Date of publication and mention of the grant of the patent: **09.07.2025**
(21) Application number: 22728236.5
(22) Date of filing: 10.05.2022
(51) Int. Cl.: C07C 29/145, C07C 31/26

(54) **PROCESS FOR THE SYNTHESIS OF L-IDITOL AND RECYCLING OF THE CATALYST**
VERFAHREN ZUR HERSTELLUNG VON L-IDITOL UND RÜCKFÜHRUNG DES KATALYSATORS
PROCÉDÉ DE SYNTHÈSE DE L-IDITOL ET RECYCLAGE DU CATALYSEUR

(30) Priority: 19.05.2021 EP 21174581
(43) Date of publication of application: 27.03.2024
(73) Proprietor: BASF SE, 67056 Ludwigshafen am Rhein (DE)
(72) Inventor: SCHAUB, Thomas, 67056 Ludwigshafen (DE); MADER, Steffen, 67056 Ludwigshafen (DE); KINDLER, Alois, 67056 Ludwigshafen (DE); HASHMI, A. Stephen K., 69117 Heidelberg (DE); TINDALL, Daniel James, 69469 Weinheim (DE)
(74) Representative: BASF IP Association
(86) International application number: PCT/EP2022/062551
(87) International publication number: WO 2022/243096

(56) References cited:
- TINDALL DANIEL J. ET AL: "Selective and Scalable Synthesis of Sugar Alcohols by Homogeneous Asymmetric Hydrogenation of Unprotected Ketoses", ANGEWANDTE CHEMIE INTERNATIONAL EDITION, vol. 60, no. 2, 11 January 2021 (2021-01-11), pages 721 - 725, XP055959953, ISSN: 1433-7851, Retrieved from the Internet <URL:https://onlinelibrary.wiley.com/doi/full-xml/10.1002/anie.202009790> DOI: 10.1002/anie.202009790

## Description

### FIELD OF THE INVENTION

The invention relates to a process for the synthesis of L-Iditol by hydrogenating L-Sorbose in a homogenous catalysed homogenous reaction and to the recycling and reactivation of the stereoselective ruthenium catalyst.

### BACKGROUND OF THE INVENTION

L-Iditol (CAS Number 488-45-9) is a sugar alcohol which can be derived by conversion of certain sugars and carbohydrates. L-Iditol can serve, inter alia, as the starting point for the synthesis of pharmaceuticals, polymers and macrocyclic compounds, but is of particular commercial interest.

As L-Iditol is only found in trace amounts in nature, isolation from natural sources is not an economic option. Therefore, the hydrogenation of L-Sorbose (CAS Number: 87-79-6) (produced as an intermediate on a large scale in the vitamin C synthesis) is of particular interest. But in the prior art, only few approaches for accessing L-Iditol from L-Sorbose have been suggested.

One way to produce L-Iditol is the reduction of L-Sorbose by fermentation with yeasts, as described by M. Ogawa et al., in Applied and Environmental Microbiology, 1983, 46 (4), 912-916. Unfortunately, the process provides only low yields of L-lditol with a maximum 33% of the consumed L-Sorbose in the fermentation, because the yeast metabolizes most of the sugar starting material under fermentation conditions. Also isolation of the L-lditol from this mixture was only possible after peracetylation, which must be followed than by a saponification of all acetyl-protecting group, to yield the pure L-Iditol in max 27 % yield and resulting in significant amounts of salt-waste from the protection/deprotection sequence.

An improved fermentation of L-Sorbitol to L-Iditol using yeasts was reported by V. Vongsuvanlert, J. Ferment. Technol. 1988, 66 (5), 517-523. Using methanol and xylose as the carbon source for the metabolism increases the yield of L-Iditol with regard to the consumed L-Sorbose up to 98 % in the crude fermentation mixture. Unfortunately, this process requires high amounts of FeSO₄ (1.12 times the amount by weight according the used sorbose) as well as strict pH-Control by using a phosphate buffer. This is a drawback, as the highly water-soluble L-Iditol must be separated from the large amount of highly water-soluble salts (which are waste) from the fermentation mixture for isolation of L-Iditol. Unfortunately no means for isolation were disclosed. Rather, only the crude fermentation mixture was analyzed by HPLC.

Principally, it should be possible to overcome the drawbacks of a fermentation by heterogeneous hydrogenation of L-Sorbose to L-Iditol. For example, EP 0 006 313A1 discloses the production of sugar alcohols by catalytic hydrogenation of keto sugars on copper catalysts containing finely divided metallic copper on a particulate support material. However, applying this process to the reduction of Sorbose yields mainly D-sorbitol. L-Iditol can only be obtained as the byproduct, with a maximum ratio of L-Iditol to D-Sorbitol of 38:62 at 100% conversion of L-Sorbose.

There is a demand that the hydrogenation can be run more selectively towards the L-Iditol, the whole process would be more efficient, for example there would no longer be a need for the subsequent fermentation as the isolation of L-Iditol from the 1:1-mixture with D-Sorbitol is very difficult and inefficient.

In Angewandte Chemie, International Edition, 60(2), 721-725 (2021) DOI: 10.1002/anie.202009790, the hydrogenation of L-Sorbose to L-Iditol with a selectivity towards L-Iditol >80% using asymmetric transition metal catalysts is described. This approach delivers for the first time in an nonenzymatic process the L-Iditol as the main product directly from L-Sorbose without the need for protection groups. However, for an efficient industrial process, a simple recycling of the used expensive stereoselective transition metal catalyst is necessary, which was not disclosed in this work.

A recycling of a stereoselective hydrogenation catalyst without a significiant loss of its activty as well as selectivty is usally not easy to achieve and in most asymmetric transformations, the catalyst is not reused.

Accordingly, it is an object of the invention to provide a process for the recycling and reactivation of a stereoselective ruthenium catalyst for the synthesis of L-Iditol by hydrogenating L-Sorbose. With this process, it should be possible to produce L-Iditol by the hydrogenation of L-Sorbose in a cost-efficient manner.

Surprisingly, it was found that the problem is solved by a process for the preparation of L-Iditol comprising at least the process steps:
i) a L-Sorbose comprising composition is subjected to hydrogenation with hydrogen in the presence of a hydrophobic stereoselective ruthenium catalyst complex in a homogeneous solution, wherein the ruthenium catalyst complex comprises at least one chiral ligand con taining at least two phosphorus atom, which are capable of coordinating to the ruthenium yielding in a composition comprising L-Iditol as the main product;
ii) separation of the reaction products produced in step i) from the ruthenium catalyst com plex;
iii) reactivating the separated ruthenium catalyst complex of step ii) by adding a chloride source and reusing the reactivated ruthenium catalyst complex in step i).

### SUMMARY OF THE INVENTION

The invention relates to a process for the preparation of L-Iditol and recycling of the stereoselective catalyst comprising the process steps: 1) a L-Sorbose comprising composition is subjected to hydrogenation with hydrogen in the presence of a hydrophobic stereoselective ruthenium catalyst complex in a homogenous solution, wherein the transition metal catalyst complex comprises at least one chiral ligand containing at least two phosphorus atoms, which are capable of coordinating to the transition metal yielding in a composition comprising L-Iditol as the main product; 2) extraction of the L-Iditol and other hexoses with water; 3) Reusing the catalyst in the selective hydrogenation of L-Sorbose to L-Iditol under addition of a chloride-source to reactivate the catalyst.

### DETAILED DESCRIPTION OF THE INVENTION

The compounds L-Sorbose, L-Iditol and D-Sorbitol have the following chemical structions depicted as Fischer projection.

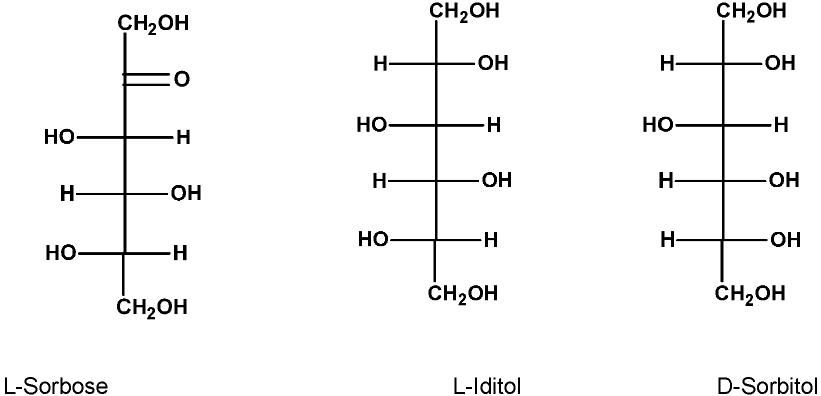

In the context of the invention, the expression "alkyl" means straight and branched alkyl groups. Preferred are straight or branched C₁-C₂₀-alkyl groups, more preferably C₁-C₁₂-alkyl groups, even more preferably C₁-C₈-alkyl groups and in particular C₁-C₆-alkyl groups. Examples of alkyl groups are particularly methyl, ethyl, propyl, isopropyl, n-butyl, isobutyl, sec-butyl, tert-butyl, n-pentyl, 2-pentyl, 2-methylbutyl, 3-methylbutyl, 1,2-dimethylpropyl, 1,1-dimethylpropyl, 2,2-dimethylpropyl, 1-ethylpropyl, n-hexyl, 2-hexyl, 2-methylpentyl, 3-methylpentyl, 4-methylpentyl, 1,2-dimethylbutyl, 1,3-dimethylbutyl, 2,3-dimethylbutyl, 1,1-dimethylbutyl, 2,2-dimethylbutyl, 3,3-dimethylbutyl, 1,1,2-trimethylpropyl, 1,2,2-trimethylpropyl, 1-ethylbutyl, 2-ethylbutyl, 1-ethyl-2-methylpropyl, n-heptyl, 2-heptyl, 3-heptyl, 2-ethylpentyl, 1-propylbutyl, n-octyl, 2-ethylhexyl, 2-propylheptyl, nonyl and decyl.

The expression "alkyl" comprises also substituted alkyl groups, which may carry 1, 2, 3, 4 or 5 substituents, preferably 1, 2 or 3 substituents and particularly preferably 1 substituent, selected from the groups cycloalkyl, aryl, hetaryl, halogen, NE¹E², NE¹E²E³⁺, COOH, carboxylate, SO₃H and sulfonate. The expression "alkyl" also comprises alkyl groups, which are interrupted by one or more non-adjacent oxygen atoms, preferably alkoxyalkyl.

The expression "alkoxy" in the context of the present invention stands for a saturated, straightchain or branched hydrocarbon radical having 1 to 4, 1 to 6, 1 to 10, 1 to 20 or 1 to 30 carbon atoms, as defined above, which is bonded via oxygen, e.g. C₁-C₄-alkoxy, such as methoxy, ethoxy, n-propoxy, 1-methylethoxy, butoxy, 1-methylpropoxy, 2-methylpropoxy or 1,1-dimethylethoxy; C₁-C₆-alkoxy: C₁-C₄-alkoxy, as specified above, and e.g. pentoxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy or 1-ethyl-2-methylpropoxy.

The expression "alkylene" in the context of the present invention stands for straight or branched alkanediyl groups having 1 to 25, preferably 1 to 6 carbon atoms. These are -CH₂-, -(CH₂)₂-, -(CH₂)₃-,-(CH₂)₄-, -(CH₂)₂-CH(CH₃)-, (-CH₂-CH(CH₃)-), -CH₂-CH(CH₃)-CH₂-, (CH₂)₄-, -(CH₂)₅-, -(CH₂)₆, -(CH₂)₇-, -CH(CH₃)-CH₂-CH₂-CH(CH₃)- or -CH(CH₃)-CH₂-CH₂-CH₂-CH(CH₃)- etc.

The expression "cycloalkyl" in the context of the present invention stands for a monocyclic, bicyclic or tricyclic, saturated hydrocarbon group having 3 to 12, preferably 3 to 6 or 3 to 8 carbon ring members, e.g. a monocyclic hydrocarbon group having 3 to 8 carbon ring members, such as cyclopropyl, cyclobutyl, cyclopentyl, cyclohexyl, cycloheptyl or cyclooctyl; a bicyclic hydrocarbon group having 5 to 10 carbon ring members, such as bicyclo[2.2.1]hept-1-yl, bicyclo[2.2.1]hept-2-yl, bicyclo[2.2.1]hept-7-yl, bicyclo[2.2.2]oct-1-yl, bicyclo[2.2.2]oct-2-yl, bicyclo[3.3.0]octyl and bicyclo[4.4.0]decyl; a tricyclic hydrocarbon group with 6 to 10 carbon ring members, such as adamantyl.

The expression "cycloalkoxy" (= cycloalkyloxy) in the context of the present invention stands for a monocyclic, bicyclic or tricyclic, saturated hydrocarbon group having 3 to 12, preferably up to 6, up to 8 carbon ring members, as defined above, which is bonded via an oxygen atom.

The expression "heterocycloalkyl" in the context of the present invention comprises saturated or partially unsaturated cycloaliphatic groups with preferably 4 to 7, more preferably 5 or 6 ring atoms, in which 1, 2, 3 or 4 ring atoms may be substituted with heteroatoms, preferably selected from the elements oxygen, nitrogen and sulfur. The heterocycloalkyl ring is optionally substituted. If substituted, these heterocycloaliphatic groups carry preferably 1, 2 or 3 substituents, more preferably 1 or 2 substituents and in particular 1 substituent. These substituents are preferably selected from alkyl, cycloalkyl, aryl, COOR (R = H, alkyl, cycloalkyl, aryl), COO⁻M⁺ and NE¹E², more preferably alkyl. Examples of such heterocycloaliphatic groups are pyrrolidinyl, piperidinyl, 2,2,6,6-tetramethylpiperidinyl, imidazolidinyl, pyrazolidinyl, oxazolidinyl, morpholidinyl, thiazolidinyl, isothiazolidinyl, isoxazolidinyl, piperazinyl, tetrahydrothiophenyl, tetrahydrofuranyl, tetrahydropyranyl and dioxanyl.

The expression "aryl" in the context of the present invention comprises a mono- or polynuclear aromatic hydrocarbon radical having usually 6 to 14, preferably 6 to 10 carbon atoms, such as e.g. phenyl, tolyl, xylyl, mesityl, naphthyl, indenyl, fluoroenyl, anthracenyl or phenanthrenyl. In case these aryl groups are substituted, they may carry preferably 1, 2, 3, 4 or 5 substituents, more preferably 1, 2 or 3 substituents and particularly preferred 1 substituent. These substituents are preferably selected from the groups alkyl, alkoxy, carboxyl, carboxylate, trifluoromethyl, -SO₃H, sulfonate, NE¹E², alkylene-NE¹E², nitro, cyano and halogen. A preferred fluorinated aryl group is pentafluorophenyl.

The expression "aryloxy" in the context of the present invention stands for a mono- or polynuclear aromatic hydrocarbon radical having usually 6 to 14, preferably 6 to 10 carbon atoms, as defined above, which is bonded via an oxygen atom.

The expression "heterocycloalkyl (= heterocyclyl) with 3 to 12 ring atoms" in the context of the present invention refers to a saturated, partially (e.g. mono-) unsaturated heterocyclic radical having 3, 4, 5, 6, 7, 8, 9, 10, 11 or 12 ring atoms, of which 1, 2 or 3 are selected from N, O, S, S(O) and S(O)₂, and the other ring atoms are carbon, such as e.g. 3- to 8-membered saturated heterocyclyl, such as oxiranyl, oxetanyl, aziranyl, piperidinyl, piperazinyl, morpholinyl, thimorpholinyl, pyrrolidinyl, oxazolidinyl, tetrahydrofuryl, dioxolanyl, dioxanyl, hexahydroazepinyl, hexyhydrooxepinyl, and hexahydrothiepinyl; partially unsaturated 3-, 4-, 5-, 6-, 7- or 8-membered heterocyclyl, such as di- and tetrahydropyridinyl, pyrrolinyl, oxazolinyl, dihydrofuryl, tetrahydroazepinyl, tetrahydrooxepinyl, and tetrahydrothiepinyl.

The expression "heterocycloalkoxy (= heterocycloalkoxy) with 3 to 12 ring atoms" in the context of the invention is a saturated, partially (e.g. mono-) unsaturated heterocyclic radical having 3, 4, 5, 6, 7, 8, 9, 10, 11 or 12 ring atoms, of which 1, 2 or 3 are selected from N, O, S, S(O) and S(O)₂, and the other ring atoms are carbon, as defined above, which is bonded via oxygen.

The expression "hetaryl (= heteroaryl)" in the context of the invention is an aromatic, mono- or polynuclear heterocycle, which, besides carbon atoms, comprises one to four heteroatoms from the group O, N or S as ring members, such as e.g.
- 5-membered heteroaryl, which, besides carbon atom(s), can comprise one to four nitrogen atoms or one to three nitrogen atoms and one sulfur or oxygen atom or one sulfur or oxygen atom as ring member, e.g. furyl, thienyl, pyrrolyl, isoxazolyl, isothiazolyl, pyrazolyl, oxazolyl, thiazolyl, imidazolyl, oxadiazolyl, thiadiazolyl, triazolyl;
- benzo-fused 5-membered heteroaryl: 5-ring heteroaryl groups as defined above, which may be condensed with one or two benzene rings in such a way that two adjacent carbon ring members or one nitrogen and one adjacent carbon ring member are bridged by a buta-1,3-diene-1,4-diyl group, e.g. indolyl, isoindolyl, benzimidazolyl, benzofuryl, benzothienyl, benzoxazolyl, benzisoxazolyl, benzothiazolyl, dibenzofuranyl, dibenzothienyl or carbazolyl;
- 6-membered heteroaryl: 6-ring heteroaryl groups, which, besides carbon atoms, can comprise one to three or one to four nitrogen atoms as ring members, e.g. pyridinyl, pyridazinyl, pyrimidinyl, pyrazinyl, 1,3,5-triazin-2-yl and 1,2,4-triazin-3-yl;
- benzo-fused 6-membered heteroaryl: 6-ring heteroaryl groups as defined above, which may be condensed with one or two benzene rings in such a way that two adjacent carbon ring members are bridged by a buta-1,3-diene-1,4-diyl group, e.g. quinolinyl, isoquinolinyl, quinazolinyl, quinoxalinyl, acridinyl or phenazinyl.

If these heterocycloaromatic groups are substituted, they may carry preferably 1, 2 or 3 substituents selected from the groups alkyl, alkoxy, carboxyl, carboxylate, -SO₃H, sulfonate, NE¹E², alkylene-NE¹E², trifluoromethyl and halogen.

Carboxylate and sulfonate in the context of the present invention preferably stand for a derivative of a carboxylic acid function or a sulfonic acid function, in particular a metal carboxylate or metal sulfonate, a carboxylic acid ester or sulfonic acid ester or a carboxylic acid amide or sulfonic acid amide. Particularly preferred are esters with C₁-C₄-alkanols like methanol, ethanol, n-propanol, isopropanol, n-butanol, sec-butanol and tert-butanol. Preferred are also the primary amides and their N-alkyl and N,N-dialkyl derivatives.

The expression "acyl" in the context of the present invention stands for alkanoyl groups or aroyl groups with preferably 2 to 11, more preferably 2 to 8 carbon atoms, for example acetyl, propanoyl, butanoyl, pentanoyl, hexanoyl, heptanoyl, 2-ethylhexanoyl, 2-propylheptanoyl, benzoyl and naphthoyl.

The groups NE¹E², NE⁴E⁵ and NE⁷E⁸ are preferably selected from N,N-dimethylamino, N,N-diethylamino, N,N-dipropylamino, N,N-diisopropylamino, N,N-di-n-butylamino, N,N-di-tert-butylamino, N,N-dicyclohexylamino and N,N-diphenylamino.

Halogen stands for fluorine, chlorine, bromine or iodine, preferably fluorine, chlorine or bromine.

Formyl ist H-C(=O)-. Carboxy is -C(=O)OH. Sulfo is -S(=O)₂-OH.

Polyalkylene oxide is a radical derived from identical or different C₂₋₄-oxyalkylene monomer building blocks, as defined above, with a degree of polymerization (number average) in the range of 2 to 100, or 3 to 50 or 4 to 25 or 5 to 10.

Polyalkyleneimine is a structure-analogous radical to the above polyalkylene oxide radical with the oxygen atom being replaced by an imine group.

M⁺ refers to a cation equivalent, which means a monovalent cation or the part of a polyvalent cation representing a positive single charge. The cation M⁺ is only a counter ion, which neutralizes negatively charged substituents like the COO⁻ or the sulfonate group and which can principally be selected arbitrarily. Preferred are alkaline metal ions, in particular Na⁺, K⁺ and Li⁺ ions, or onium ions like ammonium ions, mono-, di-, tri-, tetraalkylammonium ions, phosphonium ions, tetraalkylphosphonium ions and tetraarylphosphonium ions.

The same applies to the anion equivalent X⁻, which is only a counter ion for positively charged substituents like the ammonium group and which can principally be selected arbitrarily among monovalent anions and the parts of polyvalent anions, which correspond to a single negative charge. Preferred are halogenides X⁻, in particular chloride and bromide. Also preferred are sulfates and sulfonates, in particular SO₄²⁻, tosylate, trifluoromethane sulfonate and methylsulfonate.

Condensed ring systems, also termed fused ring systems, are aromatic, heteroaromatic or cyclic compounds, which have fused-on rings obtained via anellation. Condensed ring systems consist of two, three or more than three rings. Depending on the type of connection, one distinguishes between ortho-anellation and peri-anellation. In case of ortho-anellation, each ring has two atoms in common with each adjacent ring. In case of peri-anellation, a carbon atom belongs to more than two rings. Preferred among the condensed ring systems are ortho-condensed ring systems.

In the context of the present invention, "chiral ligands" are ligands without an axis of symmetry. They are in particular ligands with at least one chirality center (i.e. at least one asymmetric atom, in particular at least one asymmetric P atom or C atom). In a special embodiment of the present invention, addition ligands are employed, which show axial chirality. Axial chirality occurs, for example, in biphenyls, such as BINAP, which are substituted in the ortho-positions in such a way that the free rotation of the aromatic compounds around the C-C single bond is strongly hindered. This then results in two mirror-image isomers.

In the context of the present invention, the term "chiral catalyst" comprises catalysts, which have at least one chiral ligand.

"Achiral compounds" are compounds, which are not chiral.

A "prochiral compound" is understood as meaning a compound with at least one prochiral center.

"Asymmetric synthesis" refers to a reaction in which a compound with at least one chirality center is produced from a compound with at least one prochiral center, where the stereoisomeric products are formed in unequal amounts.

"Stereoisomers" are compounds of identical constitution, but different atomic arrangement in the three-dimensional space.

"Enantiomers" are stereoisomers, which behave like image to mirror image to one another. The "enantiomeric excess" (ee) achieved during asymmetric synthesis is given here by the following formula: ee [%] = (R-S)/(R+S) x 100. R and S are the descriptors of the CIP system for the two enantiomers and describe the absolute configuration on the asymmetric atom. The enantiomerically pure compound (ee = 100%) is also referred to as "homochiral compound".

The process according to the invention leads to products, which are enriched with regard to a specific stereoisomer, in particular with regard to L-Iditol. The attained "enantiomer excess" (ee) is generally at least 20%, preferably at least 50%, in particular at least 80%.

"Diastereomers" are stereoisomers, which are not enantiomeric to one another.

### Starting material

L-Sorbose is commercially available or can be prepared from D-Sorbitol via microbiological oxidation.

### Process step i):

### Catalyst

In the process of the invention, the composition comprising L-Sorbose is subjected to hydrogenation in a liquid reaction medium in the presence of a transition metal catalyst complex, which comprises at least one chiral ligand containing at least two phosphorus atoms, which are capable of coordinating to the transition metal and ruthenium as the metal center. Due to this chiral ligand the transition metal catalyst complex is stereoselective. In other words: Applying the stereoselective transition metal catalyst complex the product mixture predominately comprises the desired stereoisomer. In particular, the product mixture exclusively comprises the desired stereoisomer.

The process of the invention is carried out as a homogeneously catalyzed hydrogenation using a ruthenium catalyst complex. That means the ruthenium catalyst complex is dissolved in the liquid reaction medium under the reaction conditions. Typically, the ruthenium catalyst complex is in the same phase as the reactants, i.e. the L-Sorbose. Further, the liquid reaction medium may comprise at least one chiral ligand in excess. In this embodiment, the liquid reaction contains free chiral ligands that are not bound to the ruthenium complex. The free chiral ligands are selected from the phosphorous containing ligands defined in the following.

The ruthenium catalyst complex comprises at least one chiral ligand containing at least two phosphorus atoms, which are capable of coordinating to the transition metal. Typically, the molar ratio of the chiral ligand to the transition metal is at least 1, e.g. in the range from 1 to 4, especially 1 or 2. The chiral ligand contains at least two phosphorus atoms, which are capable of coordinating to the transition metal and is especially a chiral bidentate ligand having two phosphorous atoms, which are capable of coordinating to the ruthenium. More particularly, the ruthenium catalyst complex has 1 or 2 chiral bidentate ligands having two phosphorous atoms, which are capable of coordinating to the transition metal, in particular 1 of such a bidentate chiral ligand. Especially, the ruthenium catalyst complex has 1 or 2 chiral bidentate ligands having two phosphorous atoms, which are capable of coordinating to the transition metal, in particular 1 of such a bidentate chiral ligand.

According to the invention, the ligand containing at least two phosphorus atoms, which are capable of coordinating to the transition metal, is chiral, i.e. it bears at least one group that is asymmetric. Chirality of the ligand may be caused, e.g. because at least one of the P atoms is asymmetric, and/or the ligand has axial chirality. In particular, the chiral ligand bears a group, which causes axial chirality.

Preferably, the chiral ligand is selected from compounds of formula (I) 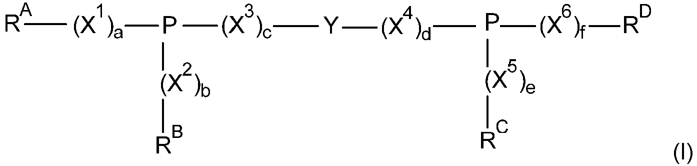 wherein
R^{A}, R^{B}, R^{C} and R^{D} are independently from each other selected from the group consisting of alkyl having in particular 1 to 30 carbon atoms, cycloalkyl having in particular 3 to 12 carbon ring members, heterocycloalkyl having in particular 3 to 12 ring atoms, aryl, such as C₆-C₁₄-aryl, and hetaryl having in particular 5 to 14 ring atoms,
wherein the alkyl radicals may be unsubstituted or carry 1, 2, 3, 4 or 5 substituents selected from cycloalkyl having in particular 5 to 8 carbon ring members, heterocycloalkyl having in particular 3 to 12 ring atoms, aryl, such as C₆-C₁₄-aryl, hetaryl having in particular 5 to 14 ring atoms, alkoxy having in particular 1 to 4 carbon atoms, cycloalkoxy having in particular 5 to 8 carbon ring members, heterocycloalkoxy having in particular 3 to 12 ring atoms, aryloxy, such as C₆-C₁₄-aryloxy, hetaryloxy having in particular 5 to 14 ring atoms, hydroxy, mercapto, polyalkylene oxide, polyalkyleneimine, carboxyl, SO₃H, sulfonate, NE¹E², NE¹E²E³⁺X⁻, halogen, nitro, formyl, acyl and cyano, wherein E¹, E² and E³ are the same or different and are selected from hydrogen, alkyl, such as C₁-C₂₀-alkyl, cycloalkyl having in particular 3 to 12 carbon ring members, and aryl, such as C₆-C₁₄-aryl, and X⁻ is an anion equivalent,
and wherein the radicals cycloalkyl, heterocycloalkyl, aryl and hetaryl in R^{A}, R^{B}, R^{C} and R^{D} may be unsubstituted or carry 1, 2, 3, 4 or 5 substituents selected from alkyl having in particular 1 to 4 carbon atoms, cycloalkyl having in particular 5 to 8 carbon ring members, heterocycloalkyl having in particular 3 to 12 ring atoms, aryl, such as C₆-C₁₄-aryl, hetaryl having in particular 5 to 14 ring atoms, alkoxy having in particular 1 to 10 carbon atoms, cycloalkoxy having in particular 5 to 8 carbon ring members, heterocycloalkoxy having in particular 3 to 12 ring atoms, aryloxy, such as C₆-C₁₄-aryloxy, hetaryloxy having in particular 5 to 14 ring atoms, hydroxy, mercapto, polyalkylene oxide, polyalkyleneimine, carboxyl, SO₃H, sulfonate, NE¹E², NE¹E²E³⁺X⁻, halogen, nitro, formyl, acyl and cyano, or
R^{A} and R^{B} and/or R^{C} and R^{D} together with the P atom and, if present, the groups X¹, X², X⁵ and X⁶ to which they are bound, are a 5- to 8-membered heterocycle, which is optionally fused with one, two or three groups selected from cycloalkyl having in particular 5 to 8 carbon ring members, heterocycloalkyl having in particular 3 to 12 ring atoms, aryl, such as C₆-C₁₄-aryl, hetaryl having in particular 5 to 14 ring atoms, wherein the heterocycle and, if present, the fused-on groups independently from each other may each carry 1, 2, 3 or 4 substituents selected from alkyl having in particular 1 to 10 carbon atoms, cycloalkyl having in particular 5 to 8 carbon ring members, heterocycloalkyl having in particular 3 to 12 ring atoms, aryl, such as C₆-C₁₄-aryl, hetaryl having in particular 5 to 14 ring atoms, hydroxy, mercapto, polyalkylene oxide, polyalkyleneimine, alkoxy, halogen, carboxyl, SO₃H, sulfonate, NE⁴E⁵, NE⁴E⁵E⁶⁺X⁻, nitro, alkoxycarbonyl, such as C₁-C₂₀-alkoxycarbonyl, formyl, acyl and cyano, wherein E⁴, E⁵ and E⁶ are the same or different and are selected from hydrogen, alkyl, such as C₁-C₂₀-alkyl, cycloalkyl having in particular 3 to 12 carbon ring members and aryl, such as C₆-C₁₄-aryl, and X⁻ is an anion equivalent,
X¹, X², X³, X⁴, X⁵, X⁶, X⁷, X⁸ and X⁹ are independently from each other O, S, CR^{x}R^{y}, SiR^{x}R^{y} or NR^{z}, wherein R^{x}, R^{y} and R^{z} are independently from each other hydrogen, alkyl having in particular 1 to 4 carbon atoms, cycloalkyl having in particular 5 to 8 carbon ring members, heterocycloalkyl having in particular 3 to 12 ring atoms, aryl, such as C₆-C₁₄-aryl, or hetaryl having in particular 5 to 14 ring atoms,
Y is a divalent bridging group, which contains carbon atoms,
a, b, c, d, e and f are independently from each other 0 or 1,
provided that formula (I) has at least one chiral group, e.g. because at least one of the P atoms is asymmetric, and/or the ligand of formula (I) has axial chirality.

In formula (I), the variables R^{A}, R^{B}, R^{C}, R^{D}, X¹, X², X³, X⁴, X⁵, X⁶, X⁷, X⁸, X⁹, a, b, c, d, e and f, individually or in particular in combination, have preferably the following meanings, provided that formula (I) has at least one chiral group, e.g. because at least one of the P atoms is asymmetric, and/or the ligand of formula (I) has axial chirality:
R^{A}, R^{B}, R^{C} and R^{D}, are independently from each other C₁-C₃₀-alkyl, C₃-C₁₂-cycloalkyl, heterocycloalkyl with 3 to 12 ring atoms, C₆-C₁₄-aryl or hetaryl with 5 to 14 ring atoms,
wherein the alkyl radical may carry 1, 2, 3, 4 or 5 substituents selected from C₃-C₁₂-cycloalkyl, heterocycloalkyl with 3 to 12 ring atoms, C₆-C₁₄-aryl, hetaryl with 5 to 14 ring atoms, C₁-C₁₀-alkoxy, C₃-C₁₂-cycloalkoxy, heterocycloalkoxy with 3 to 12 ring atoms, C₆-C₁₄-aryloxy, hetaryloxy with 5 to 14 ring atoms, hydroxy, mercapto, polyalkylene oxide, polyalkyleneimine, carboxyl, SO₃H, sulfonate, NE¹E², NE¹E²E³⁺X⁻, halogen, nitro, formyl, acyl and cyano, wherein E¹, E² and E³ are the same or different and are selected from hydrogen, C₁-C₂₀-alkyl, C₃-C₁₂-cycloalkyl, and C₆-C₁₄-aryl and X⁻ is an anion equivalent,
and wherein the radicals cycloalkyl, heterocycloalkyl, aryl and hetaryl radicals may carry 1, 2, 3, 4 or 5 substituents selected from C₁-C₂₀-alkyl and the substituents mentioned for the alkyl radical R^{A}, R^{B}, R^{C} and R^{D} before, or
R^{A} and R^{B} and/or R^{C} and R^{D} together with the P atom and, if present, the groups X¹, X², X⁵ and X⁶ to which they are bound, are a 5- to 8-membered heterocycle, which is optionally fused with one, two or three groups selected from C₃-C₁₂-cycloalkyl, heterocycloalkyl with 3 to 12 ring atoms, C₆-C₁₄-aryl and heteroaryl with 5 to 14 ring atoms, wherein the heterocycle and, if present, the fused-on groups independently from each other may each carry 1, 2, 3 or 4 substituents selected from C₁-C₂₀-alkyl, C₃-C₁₂-cycloalkyl, heterocycloalkyl with 3 to 12 ring atoms, C₆-C₁₄-aryl, hetaryl with 5 to 14 ring atoms, hydroxy, mercapto, polyalkylene oxide, polyalkyleneimine, C₁-C₂₀-alkoxy, halogen, carboxyl, SO₃H, sulfonate, NE⁴E⁵, NE⁴E⁵E⁶⁺X⁻, nitro, alkoxycarbonyl, such as C₁-C₂₀ alkoxycarbonyl, formyl, acyl and cyano, wherein E⁴, E⁵ and E⁶ are the same or different and are selected from hydrogen, C₁-C₂₀-alkyl, C₃-C₁₂-cycloalkyl and C₆-C₁₄-aryl and X⁻ is an anion equivalent,
X¹, X², X³, X⁴, X⁵, X⁶, X⁷, X⁸ and X⁹ are independently from each other O, S, CR^{x}R^{y}, SiR^{x}R^{y} or NR^{z}, wherein R^{x}, R^{y} and R^{z} are independently from each other hydrogen, C₁-C₂₀-alkyl, C₃-C₁₂-cycloalkyl, heterocycloalkyl with 3 to 12 ring atoms, C₆-C₁₄-aryl or hetaryl with 5 to 14 ring atoms,
Y is a divalent bridging group, which contains carbon atoms,
a, b, c, d, e and f are independently from each other 0 or 1.

In particular, the chiral ligand is selected from organo phosphines, in particular from compounds of the formula (I), wherein a, b, c, d, e and f are 0, or wherein X¹, X², X³, X⁴, X⁵, X⁶, X⁷, X⁸ and X⁹ are, at each occurrence, a group CR^{x}R^{y}. In particular, the integers a, b, c, d, e and f in formula (I) are 0.

In formula (I), the variables R^{A}, R^{B}, R^{C}, R^{D} have in particular the following meanings:
R^{A}, R^{B}, R^{C} and R^{D} are independently from each other selected from the group consisting of alkyl having in particular 1 to 30 carbon atoms, aryl, such as C₆-C₁₄-aryl or heteroaryl, having in particular 5 to 14 ring atoms, wherein the alkyl radical may carry 1, 2, 3, 4 or 5 substituents selected from alkoxy, such as C₁-C₈-alkoxy, NE¹E²,
NE¹E²E³⁺X⁻, wherein E¹, E² and E³ are the same or different and are selected from hydrogen or alkyl, and X⁻ is an anion equivalent, and the aryl or heteroaryl radicals may carry 1, 2, 3, 4 or 5 substituents selected from the group consisting of alkyl having in particular 1 to 8 carbon atoms, alkoxy having in particular 1 to 8 carbon atoms, NE¹E² and NE¹E²E³⁺X⁻.

More particularly, the variables R^{A}, R^{B}, R^{C}, R^{D} have in particular the following meanings:
Organo phosphines are derived from phosphines (also called phosphanes), wherein one or more hydrogens are replaced by an organic substituent.

In particular, the chiral ligand is selected from compounds of formulae (II) or (III) 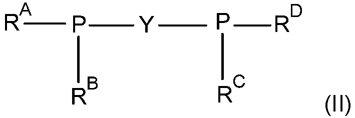 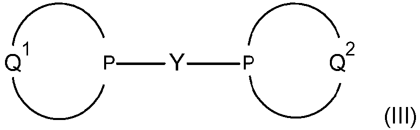 wherein,
R^{A}, R^{B}, R^{C} and R^{D} have one of the meanings as defined above,
and wherein R^{A}, R^{B}, R^{C} and R^{D} are in particular, independently of each other, selected from the group consisting of alkyl having in particular 1 to 30 carbon atoms, aryl, such as C₆-C₁₄-aryl or heteroaryl having in particular 5 to 14 ring atoms, wherein the alkyl radical may carry 1, 2, 3, 4 or 5 substituents selected from alkoxy, such as C₁-C₈-alkoxy, NE¹E², NE¹E²E³⁺X⁻, wherein E¹, E² and E³ are the same or different and are selected from hydrogen or alkyl, and X⁻ is an anion equivalent, and the aryl or heteroaryl radicals may carry 1, 2, 3, 4 or 5 substituents selected from the group consisting of alkyl having in particular 1 to 8 carbon atoms, alkoxy having in particular 1 to 8 carbon atoms, NE¹E² and NE¹E²E³⁺X⁻;
Y is a divalent bridging group, which contains carbon atoms,
Q¹, Q² and Q³ are independently from each other a divalent bridging group of the formula (IV), 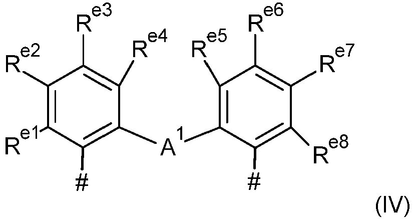
wherein
   # denotes the binding sites to the remainder of the molecule,
R^{e1} R^{e2}, R^{e3}, R^{e4}, R^{e5}, R^{e6}, R^{e7} and R^{e8} are independently from each other selected from the group consisting of hydrogen, in each case unsubstituted or substituted alkyl having in particular 1 to 20 carbon atoms, alkoxy having in particular 1 to 20 carbon atoms, cycloalkyl having in particular 3 to 12 carbon atoms, cycloalkoxy having in particular 3 to 12 carbon atoms, heterocycloalkyl having in particular 3 to 12 ring atoms, heterocycloalkoxy having in particular 3 to 12 ring atoms, aryl, such as C₆-C₁₄-aryl, aryloxy, such as C₆-C₁₄-aryloxy, hetaryl having in particular 5 to 14 ring atoms, hetaryloxy having in particular 5 to 14 ring atoms,
halogen, hydroxy, mercapto, cyano, nitro, formyl, acyl, carboxy, carboxylate, C₁-C₂₀-alkylcarbonyloxy, carbamoyl, SO₃H, sulfonate or NE⁷E⁸, wherein E⁷ and E⁸ are the same or different and are selected from hydrogen, alkyl, such as C₁-C₂₀-alkyl, cycloalkyl having in particular 3 to 12 carbon ring members, heterocycloalkyl having in particular 3 to 12 ring member atoms, aryl, such as C₆-C₁₄-aryl, and hetaryl having in particular 5 to 14 ring member atoms,
wherein two adjacent radicals R^{e1} to R^{e8} together with the carbon atoms of the benzene ring to which they are bound may also be a condensed ring system with 1, 2 or 3 further rings, and
A' is a single bond, O, S, NR^{a31}, SiR^{a32}R^{a33} or C₁-C₄-alkylene, which may have a double bond and/or which may be substituted with alkyl, such as C₁-C₂₀-alkyl, cycloalkyl having in particular 3 to 12 carbon ring members, heterocycloalkyl having in particular 3 to 12 ring member atoms, aryl, such as C₆-C₁₄-aryl, and hetaryl having in particular 5 to 14 ring member atoms, or which may be interrupted by O, S, NR^{a31} or SiR^{a32}R^{a33}, wherein R^{a31}, R^{a32} and R^{a33} are independently from each other hydrogen, alkyl, such as C₁-C₂₀-alkyl, cycloalkyl having in particular 3 to 12 carbon ring members, heterocycloalkyl having in particular 3 to 12 ring member atoms, aryl, such as C₆-C₁₄-aryl, and hetaryl having in particular 5 to 14 ring member atoms;
provided that formulae (II) and (III) have at least one chiral group, e.g. because at least one of the P atoms in formulae (II) and (III) is asymmetric and/or the ligands of formulae (II) and (III) have axial chirality.

More particularly, the chiral ligand is selected from compounds of formulae (II) or (III) 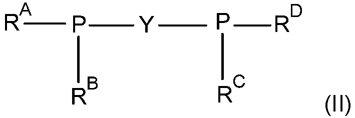 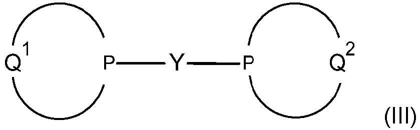 wherein
R^{A}, R^{B}, R^{C} and R^{D} have one of the meanings as defined above,
and wherein R^{A}, R^{B}, R^{C} and R^{D} are in particular, independently of each other, selected from the group consisting of alkyl having in particular 1 to 30 carbon atoms, aryl, such as C₆-C₁₄-aryl, or heteroaryl having in particular 5 to 14 ring atoms, wherein the alkyl radical may carry 1, 2, 3, 4 or 5 substituents selected from alkoxy, such as C₁-C₈-alkoxy, NE¹E², NE¹E²E³⁺X⁻, wherein E¹, E² and E³ are the same or different and are selected from hydrogen or alkyl, and X- is an anion equivalent, and the aryl or heteroaryl radicals may carry 1, 2, 3, 4 or 5 substituents selected from the group consisting of alkyl having in particular 1 to 8 carbon atoms, alkoxy having in particular 1 to 8 carbon atoms, NE¹E² and NE¹E²E³⁺X⁻;
Y is a divalent bridging group, which contains carbon atoms,
Q¹, Q² and Q³ are independently from each other a divalent bridging group of the formula (IV), 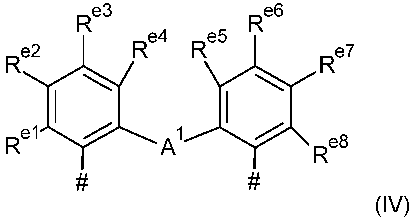
wherein
   # denotes the binding sites to the remainder of the molecule,
R^{e1}, R^{e2}, R^{e3}, R^{e4}, R^{e5}, R^{e6}, R^{e7} and R^{e8} are independently from each other hydrogen, in each case unsubstituted or substituted C₁-C₂₀-alkyl, C₁-C₂₀-alkoxy, C₃-C₁₂-cycloalkyl, C₃-C₁₂-cycloalkoxy, heterocycloalkyl with 3 to 12 ring atoms, heterocycloalkoxy with 3 to 12 ring atoms, C₆-C₁₄-aryl, C₆-C₁₄-aryloxy, hetaryl with 5 to 14 ring atoms, hetaryloxy with 5 to 14 ring atoms;
halogen, hydroxy, mercapto, cyano, nitro, formyl, acyl, carboxy, carboxylate, C₁-C₂₀-alkylcarbonyloxy, carbamoyl, SO₃H, sulfonate or NE⁷E⁸, wherein E⁷ and E⁸ are the same or different and are selected from hydrogen, C₁-C₂₀-alkyl, C₃-C₁₂-cycloalkyl, heterocycloalkyl with 3 to 12 ring atoms, C₆-C₁₄-aryl and hetaryl with 5 to 14 ring atoms,
wherein two adjacent radicals R^{e1} to R^{e8} together with the carbon atoms of the benzene ring to which they are bound may also be a condensed ring system with 1, 2 or 3 further rings, and
A' is a single bond, O, S, NR^{a31}, SiR^{a32}R^{a33} or
C₁-C₄-alkylene, which may have a double bond and/or C₁-C₄-alkylene which may be substituted with C₁-C₂₀-alkyl, C₃-C₁₂-cycloalkyl, heterocycloalkyl with 3 to 12 ring atoms, C₆-C₁₄-aryl or hetaryl with 5 to 14 ring atoms or C₁-C₄-alkylene which may be interrupted by O, S, NR^{a31} or SiR^{a32}R^{a33}, wherein R^{a31}, R^{a32} and R^{a33} are independently from each other hydrogen, C₁-C₂₀-alkyl, C₃-C₁₂-cycloalkyl, heterocycloalkyl with 3 to 12 ring atoms, C₆-C₁₄-aryl or hetaryl with 5 to 14 ring atoms, provided that formulae (II) and (III) have at least one chiral group, e.g. because at least one of the P atoms in formulae (II) and (III) is asymmetric and/or the ligands of formulae (II) and (III) have axial chirality.

In formula (IV), the radicals R^{e1} R^{e2}, R^{e3}, R^{e4}, R^{e5}, R^{e6}, R^{e7} and R^{e8} are preferably independently from each other selected from the group consisting of hydrogen, halogen in each case unsubstituted or substituted C₁-C₁₀-alkyl, C₁-C₁₀-alkoxy, C₆-C₁₄-aryl, hetaryl with 5 to 10 atoms, or two adjacent radicals R^{e1} to R^{e8} together with the carbon atoms of the benzene ring to which they are bound may also be a condensed ring system with one further ring, and
in formula (IV) the radical A' is in particular a single bond, O or S.

In a very preferred group of embodiments, the transition metal catalyst complex comprises at least one chiral ligand selected from compounds of formulae (I) or (II), wherein
R^{A}, R^{B}, R^{C} and R^{D} are independently from each other alkyl having in particular 1 to 30 carbon atoms, aryl, such as C₆-C₁₄-aryl, or heteroaryl having in particular 5 to 14 ring atoms, wherein the alkyl radical may carry 1, 2, 3, 4 or 5 substituents selected from alkoxy, NE¹E², NE¹E²E³⁺X⁻, wherein E¹, E² and E³ are the same or different and are selected from hydrogen or alkyl having in particular 1 to 10 carbon atoms, and X- is an anion equivalent, and wherein the aryl or heteroaryl radicals may carry 1, 2, 3, 4 or 5 substituents selected from alkyl, alkoxy, NE¹E², NE¹E²E³⁺X⁻, wherein E¹, E² and E³ are the same or different and are selected from hydrogen or alkyl having in particular 1 to 10 carbon atoms, and X⁻ is an anion equivalent,
Y is a divalent bridging group, which contains carbon atoms, and
a, b, c, d, e and f are independently from each other 0,
provided that formulae (I) and (II) have at least one chiral group, e.g. because at least one of the P atoms in formulae (I) and (II) is asymmetric, and/or the ligands of formulae (I) and (II) have axial chirality.

Even more preferably, the transition metal catalyst complex comprises at least one chiral ligand selected from compounds of formulae (I) or (II), wherein
R^{A}, R^{B}, R^{C} and R^{D} are independently from each other C₁-C₁₀-alkyl, C₆-C₁₂-aryl or heteroaryl with 5 to 10 ring atoms, wherein the alkyl radical may carry 1, 2, 3, 4 or 5 substituents selected from C₁-C₁₀-alkoxy, NE¹E², NE¹E²E³⁺X⁻, wherein E¹, E² and E³ are the same or different and are selected from hydrogen or C₁-C₁₀-alkyl, and X⁻ is an anion equivalent, and wherein the aryl or heteroaryl radicals may carry 1, 2, 3, 4 or 5 substituents selected from C₁-C₁₀-alkyl, C₁-C₁₀-alkoxy, NE¹E², NE¹E²E³⁺X⁻, wherein E¹, E² and E³ are the same or different and are selected from hydrogen or C₁-C₁₀-alkyl, and X⁻ is an anion equivalent,
Y is a divalent bridging group, which contains carbon atoms, and
a, b, c, d, e and f are independently from each other 0,
provided that formulae (I) and (II) have at least one chiral group, e.g. because at least one of the P atoms in formulae (I) and (II) is asymmetric, and/or the ligands of formulae (I) and (II) have axial chirality.

Especially, the transition metal catalyst complex comprises at least one ligand selected from compounds of formulae (I) or (II), wherein
R^{A}, R^{B}, R^{C} and R^{D} are C₆-C₁₂-aryl, especially phenyl, which may carry 1, 2, 3, 4 or 5 substituents selected from C₁-C₁₀-alkyl and C₁-C₁₀-alkoxy,
Y is a divalent bridging group, which contains carbon atoms, and
a, b, c, d, e and f are independently from each other 0,
provided that formulae (I) and (II) have at least one chiral group, e.g. because at least one of the P atoms in formulae (I) and (II) is asymmetric, and/or the ligands of formulae (I) and (II) have axial chirality.

As mentioned above, the chiral ligand either contains at least one chirality center or exhibit axial chirality.

In a preferred embodiment, the chiral ligand exhibits axial chirality. Especially, the ligands of formulae (I), (II) and (III) have axial chirality. Especially, axial chirality is caused by the bridging group Y.

Preferably, the divalent bridging group Y in formulae (I), (II) and (III) is selected from groups of the formulae (V) or (VI): 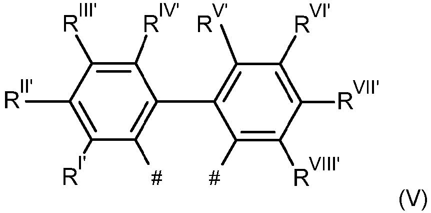 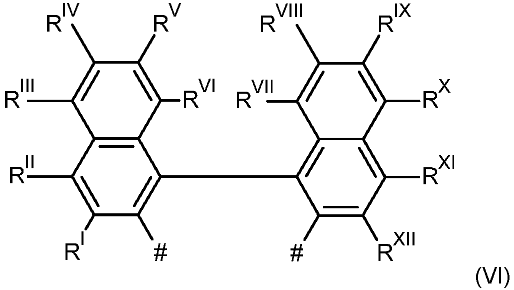 wherein
# denotes the binding sites to the remainder of the molecule,
R^{I}, R^{I'}, R^{II}, R^{II'}, R^{III}, R^{III'}, R^{IV}, R^{IV'}, R^{V}, R^{V'}, R^{VI}, R^{VI'}, R^{VII}, R^{VII'}, R^{VIII'}, R^{VIII}, R^{IX}, R^{X}, R^{XI} and R^{XII} are each, independently from each other, hydrogen, alkyl having in particular 1 to 20 carbon atoms, cycloalkyl having in particular 3 to 12 carbon ring members, heterocycloalkyl having in particular 3 to 12 ring atoms, aryl, such as C₆-C₁₄-aryl, and hetaryl having in particular 5 to 14 ring atoms, hydroxy, thiol, polyalkylene oxide, polyalkylenimine, alkoxy, halogen, SO₃H, sulfonate, NE¹E², alkylene-NE¹E², nitro, alkoxycarbonyl, carboxyl, acyl or cyano, wherein E¹ and E² are as defined above and in particular selected from the group consisting of hydrogen, alkyl having in particular 1 to 20 carbon atoms, cycloalkyl having in particular 3 to 12 carbon ring members and aryl, such as C₆-C₁₄-aryl,
wherein two adjacent radicals R^{I'}, R^{II'}, R^{III'}, R^{IV'}, R^{V'}, R^{VI'}, R^{VII'}, R^{VIII'} together with the carbon atoms of the benzene ring to which they are bound may also be a condensed ring system with 1, 2 or 3 further rings, wherein the ring atoms are selected from carbon, oxygen and sulfur, and wherein each of the rings may carry 1, 2 or 3 substituents selected from halogen, C₁-C₄-alkyl and C₁-C₄-alkoxy,
wherein two radicals R^{IV'} and R^{V'} together with the carbon atoms of the two benzene rings to which they are bound may also be a condensed ring system, wherein the ring atoms are selected from carbon, oxygen and sulfur, and wherein each of the rings may carry 1, 2 or 3 substituents selected from halogen C₁-C₄-alkyl and C₁-C₄-alkoxy.

More preferably, the divalent bridging group Y has one of the meanings of formulae (V) or (VI) 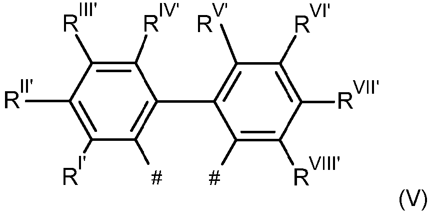 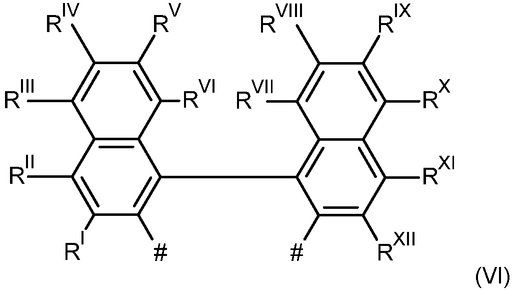 wherein
# denotes the binding sites to the remainder of the molecule,
R^{I}, R^{I'}, R^{II}, R^{II'}, R^{III}, R^{III'}, R^{IV}, R^{IV'}, R^{V}, R^{V'}, R^{VI}, R^{VI'}, R^{VII}, R^{VII'}, R^{VIII'}, R^{VIII}, R^{IX}, R^{X}, R^{XI} and R^{XII} are each, independently from each other, hydrogen, C₁-C₂₀-alkyl, C₃-C₁₂-cycloalkyl, heterocycloalkyl with 3 to 12 ring atoms, C₆-C₁₄-aryl, hetaryl with 5 to 14 ring atoms, hydroxy, thiol, polyalkylene oxide, polyalkylenimine, C₁-C₂₀-alkoxy, halogen, SO₃H, sulfonate, NE¹E², alkylene-NE¹E², nitro, C₁-C₂₀-alkoxycarbonyl, carboxyl, acyl or cyano, wherein E¹ and E² are identical or different and are selected from hydrogen, C₁-C₂₀-alkyl, C₃-C₁₂-cycloalkyl and C₆-C₁₄-aryl,
wherein two adjacent radicals R^{I'}, R^{II'}, R^{III'}, R^{IV'}, R^{V'}, R^{VI'}, R^{VII'}, R^{VIII'} together with the carbon atoms of the benzene ring to which they are bound may also be a condensed ring system with 1, 2 or 3 further rings, wherein the ring atoms are selected from carbon, oxygen and sulfur, and
wherein each of the rings may carry 1, 2 or 3 substituents selected from halogen, C₁-C₄-alkyl and C₁-C₄-alkoxy,
wherein two radicals R^{IV'} and R^{V'} together with the carbon atoms of the two benzene rings to which they are bound may also be a condensed ring system, wherein the ring atoms are selected from carbon, oxygen and sulfur, and wherein each of the rings may carry 1, 2 or 3 substituents selected from halogen C₁-C₄-alkyl and C₁-C₄-alkoxy.

Especially, the radicals R^{I}, R^{I'}, R^{II}, R^{II'}, R^{III}, R^{III'}, R^{IV}, R^{IV'}, R^{V}, R^{V'}, R^{VI}, R^{VI'}, R^{VII}, R^{VII'}, R^{VIII'}, R^{VIII}, R^{IX}, R^{X}, R^{XI} and R^{XII} are each, independently from each other, hydrogen, C₁-C₁₀-alkyl, C₆-C₁₂-aryl, hetaryl with 5 to 10 atoms,
wherein two adjacent radicals R^{I'}, R^{II'}, R^{III'}, R^{IV'}, R^{V'}, R^{VI'}, R^{VII'}, R^{VIII'} together with the carbon atoms of the benzene ring to which they are bound may also be a condensed ring system with 1, 2 or 3 further rings, wherein the ring atoms are selected from carbon, oxygen and sulfur, and wherein the each of the rings may carry 1, 2 or 3 substituents selected from halogen, C₁-C₄-alkyl and C₁-C₄-alkoxy,
wherein two radicals R^{IV'} and R^{V'} together with the carbon atoms of the benzene ring to which they are bound may also be a condensed ring system, wherein the ring atoms are selected from carbon, oxygen and sulfur, and wherein each of the rings may carry 1, 2 or 3 substituents selected from halogen C₁-C₄-alkyl and C₁-C₄-alkoxy.

Especially, the divalent bridging group Y has one of the meanings of formulae (V), wherein R^{I}, R^{I'}, R^{II}, R^{II'}, R^{III}, R^{III'}, R^{IV} and R^{IV'} are each, independently from each other, hydrogen, C₁-C₁₀-alkyl, C₆-C₁₂-aryl, hetaryl with 5 to 10 atoms,
wherein two adjacent radicals R^{I'}, R^{II'}, R^{III'}, R^{IV'}, R^{V'}, R^{VI'}, R^{VII'}, R^{VIII'} together with the carbon atoms of the benzene ring to which they are bound may also be a condensed ring system with 1, 2 or 3 further rings, wherein the ring atoms are selected from carbon, oxygen and sulfur, and wherein each of the rings may carry 1, 2 or 3 substituents selected from halogen, C₁-C₄-alkyl and C₁-C₄-alkoxy,
wherein two radicals R^{IV'} and R^{V'} together with the carbon atoms of the benzene ring to which they are bound may also be a condensed ring system, wherein the ring atoms are selected from carbon, oxygen and sulfur, and wherein each of the rings may carry 1, 2 or 3 substituents selected from halogen C₁-C₄-alkyl and C₁-C₄-alkoxy.

The ligand is selected in a manner, that a catalyst is formed, which has a low solubility in water. Preferably it is selected in way, that in the process step 3 the amount of ruthenium in the aqueous phase after the extraction is below 1 part per million. The below given ligands A-H will result in a ruthenium catalyst, which provides the required low solubility of the ruthenium catalyst in water.

In especially preferred embodiments, the ruthenium catalyst complex comprises at least one ligand selected from the formulae A to H and mixtures thereof

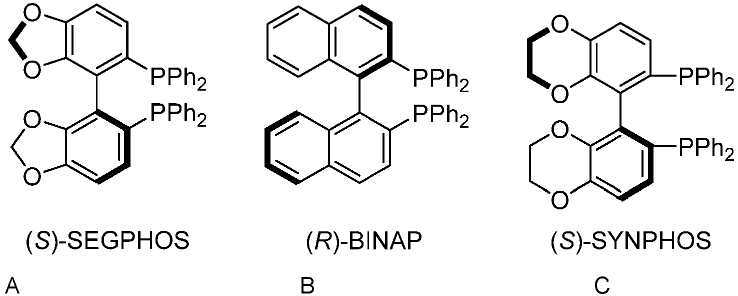

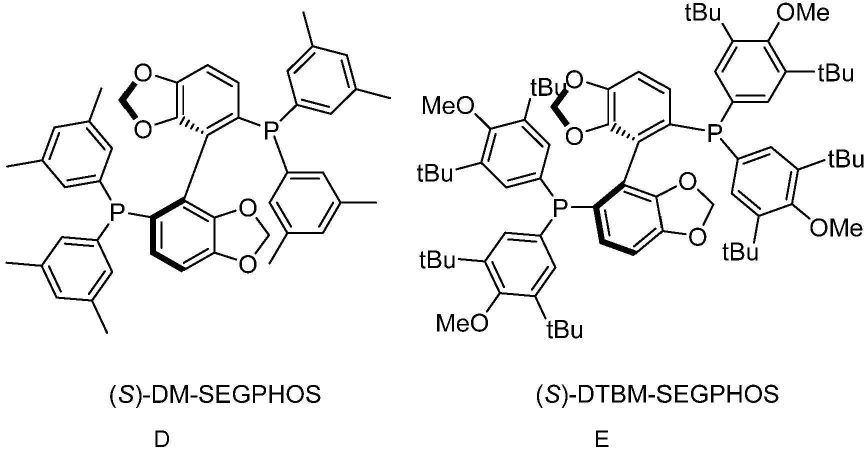

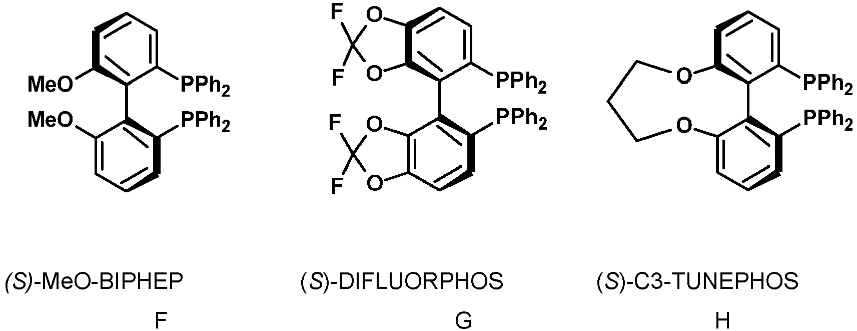

### Abbreviations:

- Me: methyl
- Ph: phenyl
- ^{t}Bu: tert butyl

- (S)-SEGPHOS: (S)-(-)-5,5'-Bis(diphenylphosphino)-4,4'-bi-1,3-benzodioxole
- (R)-BINAP: (*R*)-(+)-(1,1'-Binaphthalene-2,2'-diyl)bis(diphenylphosphine)
- (S)-SYNPHOS: (S)-6,6'-Bis(diphenylphosphino)-2,2',3,3'-tetrahydro-5,5'-bibenzo[b][1,4]dioxine
- (S)-DM-SEGPHOS: (S)-(-)-5,5'-Bis(diphenylphosphino)-4,4'-bi-1,3-benzodioxole
- (S)-DTBM-SEGPHOS: (S)-(+)-5,5'-Bis[di(3,5-di-tert-butyl-4-methoxyphenyl)phosphino]-4,4'-bi-1,3-benzodioxole
- (S)-MeO-BIPHEP: (S)-(-)-2,2'-Bis(diphenylphosphino)-6,6'-dimethoxy-1,1'-biphenyl
- (S)-DIFLUORPHOS: S-(+)-5,5'-Bis(diphenylphosphino)-2,2,2',2'-tetrafluoro-4,4'-bi-1,3-benzodioxole
- (S)-C3-TUNEPHOS: (S)-Bis(diphenylphosphino)-7,8-dihydro-6H-dibenzo[f,h][1,5]dioxonin

The ruthenium catalyst according to the invention can be employed in the form of a preformed complex, which comprises the ruthenium compound and one or more ligands. Alternatively, the ruthnium catalyst is formed in situ in the reaction medium by combining a metal compound, herein also termed pre-catalyst, with one or more suitable ligands to form a catalytically ruthenium complex in the reaction medium. It is also possible that the ruthenium catalyst is formed in situ in the presence of an auxiliary ligand by combining a metal compound, herein also termed pre-catalyst, with one or more auxiliary ligands to form a catalytically ruthenium complex in the reaction medium.
Suitable pre-catalysts are selected from neutral ruthenium complexes, oxides and salts of ruthenium

Ruthenium compounds that are useful as pre-catalyst are, for example, [Ru(methylallyl)₂COD], [Ru(p-cymene)Cl₂]₂, [Ru(benzene)Cl₂]ₙ, [Ru(CO)₂Cl₂]ₙ, [Ru(CO)₃Cl₂]₂, [Ru(COD)(allyl)], [RuCl₃·H₂O], [Ru(acetylacetonate)₃], [Ru(DMSO)₄Cl₂], [Ru(PPh₃)₃(CO)(H)Cl], [Ru(PPh₃)₃(CO)Cl₂], [Ru(PPh₃)₃(CO)(H)₂], [Ru(PPh₃)₃Cl₂], [Ru(Cp)(PPh₃)₂Cl], [Ru(Cp) (CO)₂Cl], [Ru(Cp)(CO)₂H], [Ru(Cp)(CO)₂]₂, [Ru(Cp*)(CO)₂Cl], [Ru(Cp*)(CO)₂H], [Ru(Cp*)(CO)₂]₂, [Ru(indenyl)(CO)₂Cl], [Ru(indenyl)(CO)₂H], [Ru(indenyl)(CO)₂]₂, ruthenocen, [Ru(binap)(Cl)₂], [Ru(2,2'-bipyridin)₂(Cl)₂-H₂O], [Ru(COD)(Cl)₂H]₂, [Ru(Cp*)(COD)Cl], [Ru₃(CO)₁₂], [Ru(tetraphenylhydroxycyclopentadienyl)(CO)₂H], [Ru(PMe₃)₄(H)₂], [Ru(PEt₃)₄(H)₂], [Ru(Pn-Pr₃)₄(H)₂], [Ru(P*n*-Bu₃)₄(H)₂], [Ru(P*n*-octyl₃)₄(H)₂], of which [Ru(methylallyl)₂COD], Ru(COD)Cl₂]₂, [Ru(P*n*-Bu₃)₄(H)₂], [Ru(P*n*-octyl₃)₄(H)₂], [Ru(PPh₃)₃(CO)(H)Cl] and [Ru(PPh₃)₃(CO)(H)₂] are preferred, in particular [Ru(methylallyl)₂COD].

In the aforementioned compound, names "COD" denotes 1,5-cyclooctadiene; "Cp" denotes cyclopentadienyl; "Cp*" denotes pentamethylcycopentadienyl; and "binap" denotes 2,2'-bis(diphenylphosphino)-1,1'-binaphthyl.

In the process of the invention, a sub-stoichiometric amount of the catalyst is generally used with the amount of catalyst typically being not more than 50 mol%, frequently not more than 20 mol% and in particular not more than 10 mol% or not more than 5 mol%, based on the amount of L-Sorbose in the L-Sorbose comprising composition. An amount of catalyst of from 0.001 to 50 mol%, frequently from 0.001 mol% to 20 mol% and in particular from 0.005 to 5 mol%, based on the amount of L-Sorbose in the L-Sorbose comprising composition, is generally used in the process of the invention. Preference is given to using an amount of catalyst of from 0.01 to 2 mol% and particularly preferably from 0.01 mol% to 1 mol%. All amounts of catalysts indicated are calculated as ruthenium metal and based on the amount of L-Sorbose in the L-Sorbose comprising composition.

Typically, the amount of the chiral ligand present in the process of the invention is at least 0.5 mol, in particular at least 0.8 mol, especially at least 1 mol per 1 mol of ruthenium metal, e.g. in the range of 0.5 to 10 mol, in particular in the range of 0.8 to 8 mol and especially in the range from 1.0 to 5.0 mol per 1 mol of the transition metal.

The process of the invention can be carried out in the presence of a solvent. Suitable solvents are selected from aliphatic hydrocarbons, aromatic hydrocarbons, amides, ureas, nitriles, sulfoxides, sulfones, alcohols, esters, carbonates, ethers and mixtures thereof. Preferred solvents are
- aliphatic and alicyclic hydrocarbons, in particular those having 5 to 10 carbon atoms, such as pentane, hexane, heptane, octane, cyclohexane and methylcyclohexane;
- aromatic hydrocarbons including halogen containing aromatic hydrocarbons, such as benzene, toluene, xylenes, ethylbenzene, mesitylene or benzotrifluoride;
- amides, in particular N,N-dialkylamides of aliphatic carboxylic acids and N-alkyllactams, such as as dimethylformamide, diethylformamide, N-methylpyrrolidone, N-ethylpyrrolidone or dimethylacetamide;
- ureas, in particular N,N,N',N'-tetraalkyl ureas and N,N'-dialkyl-N,N'-alkylene ureas, such as tetramethylurea, N,N-dimethylimidazolinone (DMI) and N,N-dimethylpropyleneurea (DMPU);
- nitriles, in particular aliphatic nitriles, such as acetonitrile or propionitrile;
- sulfoxides, in particular dialkylsulfoxide, such as dimethyl sulfoxide;
- sulfones, in particular alicyclic sulfones, such as sulfolane;
- alcohols, in particular alkanols, such as methanol, ethanol, propanol, isopropanol, 1-butanol, iso-butanol, 1-propanol, iso-propanol, 1-hexanol;
- esters, in particular alkyl esters of aliphatic carboxylic acids, such as methyl acetate, ethyl acetate, t-butyl acetate and ethylbutyrate;
- carbonates, in particular dialkyl carbonates and alkylene carbonates, such as diethyl car bonate, ethylene carbonate and propylene carbonate;
- ethers, in particular dialkyl ethers and alicyclic ethers, such as dioxane, tetrahydrofurane, diethyl ether, dibutyl ether, methyl t-butyl ether, diisopropyl ether or diethylene glycol di methyl ether;

If desired, mixtures of two or more of the aforementioned solvents can also be used.

In a preferred embodiment, preferred solvent are alcohols, in particular C₁-C₈-alkanols, such as methanol, ethanol, propanol, isopropanol, 1-butanol, iso-butanol, 1-propanol, iso-propanol, 1-hexanol or mixtures thereof.

### Process step i)

The hydrogenation can principally be performed according to all processes known to a person skilled in the art, which are suitable for the hydrogenation of a L-Sorbose comprising composition.

The hydrogen used for the hydrogenation can be used in pure form or, if desired, also in the form of mixtures with other, preferably inert gases, such as nitrogen or argon. Preference is given to using hydrogen in undiluted form.

The hydrogenation is typically carried out at a hydrogen pressure in the range from 0.1 to 300 bar, preferably in the range from 1 to 100 bar, more preferably in the range from 1 to 50 bar.

The hydrogenation is typically carried out at a temperature in the range from -20 to 300°C. The hydrogenation is preferably carried out at a temperature of at least 50°C, in particular at least 80°C. Preferably, the temperature will not exceed 200°C, in particular 180°C. The hydrogenation is in particular carried out at a temperature in the range of 50°C to 200°C and particularly preferably in the range from 80°C to 180°C. Temperatures of at most 150°C, e.g. in the range from 50 to 150°C, in particular in the range from 80 to 150°C, are particularly advantageous.

The hydrogenation can principally be performed in all reactors known by a person in the art for this type of reaction, and, therefore, will select the reactors accordingly. Suitable reactors are described for example in "Ullmanns Enzyklopädie der technischen Chemie", Vol. 1, 3rd edition, 1951, page 743 ff. Suitable pressure-resistant reactors are also known to a person skilled in the art and are described, for example, in "Ullmanns Enzyklopädie der technischen Chemie", Vol. 1, 3rd edition, 1951, page 769 ff. Preferably, for the hydrogenation an autoclave is employed, which may have an internal stirrer and an internal lining.

In a preferred embodiment according to the invention, the obtained composition comprises L-Iditol as the main product and D-Sorbitol as the minor compound. The obtained composition is enriched in L-Iditol and depleted in L-Sorbose, D-Mannitol and D-Sorbitol. The ratio of D-Sorbitol to L-Iditol is in the range of 1:7 to 1:1.5, preferably in the range of 1:6.5 to 1:1.9. Besides L-Iditol and other hexitols, the reaction mixture also contains the solvent and the stereoselective ruthenium catalyst in the form of its resting state.

### Process step ii)

From the reaction mixture obtained in step i), the L-Iditol as the main product as well as the other hexoses were separated from the catalyst system. This separation is preferably performed as an extraction with water. The amount of water used is in a range of 3 to 100 mass equivalents according the L-Iditol present in the residue obtained in step ii), preferably 5 to 15 mass equivalents according the L-Iditol present in the residue obtained in step ii). If a solvent was used in the hydrogenation reaction (step i)) providing a mixing gap with water, the L-Iditol can be directly extracted from the reaction mixture. The extraction can be carried out in any state-of-the art extraction apparatus like mixer-settler. Another way to extract the products can be selective membranes, where only the hexitols can permeate and not the ruthenium catalyst. By this extraction, the L-Iditol as the main product and the other hexoses as minor products will be dissolved in the water, whereas the resting state of the ruthenium catalyst with a ligand as defined above will remain in the non-polar phase with a ruthenium concentration in the aqueous phase of not more than 1 ppm.

If the solvent used in step i) provides no mixing gap with water, like methanol, the products can also be extracted by first removing the solvent by distillation or in vacuo leaving a residue, from which the L-Iditol as the main product as well as the other hexoses were extracted with water. This extraction can be performed in any setting used in the state of the art for extraction, like a stirred reactor, stirred tank, Soxhlet or filtration unit. In this step, the L-Iditol as the main product and the other hexoses as minor products will be dissolved in the water, whereas the resting state of the ruthenium catalyst with a ligand as defined above will remain as insoluble solid with a ruthenium concentration in the aqueous phase of not more than 1 ppm. the remaining solid ruthenium catalyst in the form of its resting state is separated from the aqueous by any setting used in the state of the art for the separation of a liquid and solid like filtration, decanting or centrifugation.

From the separated water phase, the L-Iditol as the main product and the other hexoses as minor product can be obtained by evaporating of the water and used as obtained or further purified by state-of-the-art methods, if necessary.

### Process step iii)

The separated catalyst in the form of its resting state obtained in step ii) is reactivated by adding a chloride source to the catalyst.

The chloride source can be HCl or a chloride salt or anion-exchange resins in a Cl-form. Preferred chloride salts are LiCl, NaCl, KCI, CaCl₂, MgCl₂, AlCl₃, FeCl₃. Preference is given to HCl as a methanolic solution or as HCl gas.

The amount of chloride used is in the range of 1 to 50 molar equivalent according the amount of ruthenium in the recycled catalyst, preferable in a range of 1 to 5 equivalents chloride according the ruthenium.

After the reactivation of the catalyst according to step iii) the catalyst can be reused in the hydrogenation reaction (step i)), either immediately after step iii) or after a storage period. All process steps can either be run in a continuous- or in a discontinuous manner.

Without adding a chloride source, the activity and selectivity of the recycled catalyst is low (see comparative example).

Furthermore the use of a transition metal complex, defined by a hydrophobic stereoselective ruthenium catalyst complex comprising at least one chiral ligand containing at least two phosphorus atoms, which are capable of coordinating to the ruthenium, represented by formula (I), wherein Y in formula (I) is a divalent bridging group, which contains carbon atoms, and Y being preferably selected from groups of formulae (V) or (VI), as hydrogenation catalyst for the hydrogenation of compositions comprising L-sorbose or mixtures thereof, was found, characterized in that
(i) the hydrogenation is performed with hydrogen in homogeneous solution
(ii) the reaction products produced in step i) are separated from the ruthenium catalyst complex, and
(iii) the separated ruthenium catalyst complex of step ii) is reactivated by adding a chloride source and reused in step i).

The invention is described in more detail in the following examples.

### EXAMPLES

All chemicals and solvents were purchased from Sigma-Aldrich, Merck or ABCR and were used without further purification.

Analytics of the reaction mixture after hydrogenation:
Samples of carbohydrates, e.g. hexoses, pentoses and, after a suitable hydrogenation, the corresponding sugar alcohols, were diluted in water to obtain a mass concentration of approximately 200 mg/ml prior to high-performance liquid chromatographic (HPLC) separation. Compositional analysis of the said samples was performed by the means of ion-moderated partition chromatography using refractive index detection. Known signals were quantified by external standard quantification. Separation was achieved using two serially coupled 300 mm x 7.8 mm Aminex HPX-87P columns (Bio-Rad Laboratories). Separation took place after injecting an aliquot of the sample into the HPLC system using deionized water as mobile phase at a column temperature of 80°C.

### Example 1:

First Hydrogenation: In an argon-filled glovebox, a 180 mL Premex stainless steel autoclave fitted with a Teflon insert was charged with L-sorbose (7.35 g, 40.8 mmol), [RuCl₂(benzene)]₂ (0.109 g, 0.217 mmol), Ligand E (0.531 g, 0.450 mmol), MeOH (80 mL), and a stir bar. After the autoclave was closed, the reaction vessel was removed from the glovebox. The system was purged twice with hydrogen (20 bar) and then pressurized with hydrogen (60 bar, ca. 5 min) and placed into a preheated heating block (100 °C) over a magnetic stirring plate. While stirring overnight the pressure reached ca. 55 bar at the reaction temperature. After 12 h, the autoclave was then placed in a cold water bath, and after it was cooled to room temperature, it was depressurized.

The golden-yellow solution was transferred to a round-bottom flask. The solution was evaporated and dried in vacuo to yield a yellow-brown sticky residue.

The rest of the residue was mixed with distilled water (50 mL) and the light brown suspension was filtered over a fritted filter (∅ 3.5 cm, pore size 4) and then over a pad of celite (∅ 3.5 cm, 7-10 mm high) sitting on a fritted filter (∅ 3.5 cm, pore size 4). A sample (1 mL) of the lime-like colored solution was submitted to ICP-MS analysis. The ruthenium-content in this solution was 1 mg/kg as determined by ICP/MS.

The brown filter cake was washed with distilled water (2 × 10 mL) and dried in vacuo to yield 537.7 mg of the ruthenium catalyst in its resting state. These two combined aqueous fractions were filtered over a pad of celite (∅ 3.5 cm, 7-10 mm high) sitting on a fritted filter (∅ 3.5 cm, pore size 4) and added to the above-mentioned aqueous fraction. The solution was dried using the cryovap method (*OPRD* **2020,** *24*, 25) followed by drying on the high vacuum over the weekend resulting in a highly viscous lime-colored liquid (7.80 g). According HPLC analysis of this viscous lime-colored liquid, the conversion of L-Sorbose was 99% and the overall selectivity towards L-Iditol and D-Sorbitol was 98%. The ratio between L-Iditol to D-Sorbitol was 6.7 to 1, according a L-Iditol yield of 84.4 % after the first hydrogenation.

Second Hydrogenation with recycled catalyst and HCl addition:
In an argon-filled glovebox, a 60 mL Premex stainless steel autoclave fitted with a Teflon insert was charged with L-Sorbose (730 mg, 4.05 mmol), the catalyst obtained after the product extraction from the first hydrogenation (62 mg), MeOH (8 mL), 0.5 M methanolic HCl (0.16 mL, 0.08mmol), and a stir bar. After it was closed, the reaction vessel was removed from the glovebox and placed into a preheated heating block (100 °C) over a magnetic stirring plate. After 16h, the autoclave was then placed in a cold water bath, and after it was cooled to room temperature, it was depressurized.

The volatiles of a sample (2 mL) of the brown solution were evaporated to dryness. The residue was dissolved in distilled water (2 mL). The suspension was filtered over a pad of celite and subsequently through a PTFE syringe filter (0.2 µm) and submitted to HPLC analysis. According HPLC, the conversion of L-Sorbose was 98% and the overall selectivity towards L-Iditol and D-Sorbitol was 96%. The ratio between L-Iditol to D-Sorbitol was 6.4 to 1, according a L-Iditol yield of 84.4 % after the first hydrogenation.

### Comparative Example 1:

Second Hydrogenation with recycled catalyst but without HCl addition:
In an argon-filled glovebox, a 60 mL Premex stainless steel autoclave fitted with a Teflon insert was charged with L-Sorbose (736.2 mg, 4.09 mmol), the catalyst after the product extraction from the first hydrogenation of example 1 (74.0 mg), MeOH (8 mL), and a stir bar (15:05). After it was closed, the reaction vessel was removed from the glovebox and placed into a preheated heating block (100 °C) over a magnetic stirring plate. After 16 h, the autoclave was then placed in a cold water bath, and after it was cooled to room temperature, it was depressurized.

The solvent of the yellow-brown solution was evaporated and the brown residue was dissolved in dest. water (8 mL). The light brown suspension was subjected to centrifugation (40,000 rpm, 30 min). The supernatant was filtered through a PTFE syringe filter (0.2 µm) and submitted to HPLC analysis. According HPLC, the conversion of L-Sorbose was 88% and the overall selectivity towards L-Iditol and D-Sorbitol was 91%. The ratio between L-Iditol to D-Sorbitol was 1.8 to 1, according a L-Iditol yield of 51.5 % after the first hydrogenation. This comparative experiment shows, that without adding a chloride source, the activity and selectivity significantly drops when the catalyst is recycled.

## Claims

1. A process for the preparation of L-Iditol comprising at least the process steps:
i) a L-Sorbose comprising composition is subjected to hydrogenation with hydrogen in the presence of a hydrophobic stereoselective ruthenium catalyst complex in a homogeneous solution, wherein the ruthenium catalyst complex comprises at least one chiral ligand containing at least two phosphorus atom, which are capable of coordinating to the ruthenium yielding in a composition comprising L-Iditol as the main product;
ii) separation of the reaction products produced in step i) from the ruthenium catalyst complex ;
iii) reactivating the separated ruthenium catalyst complex of step ii) by adding a chloride source and reusing the reactivated ruthenium catalyst complex in step i).

2. A process according to claim 1, wherein the separation of the reaction products in step ii) is performed as an extraction with water.

3. A process according to claim 1, wherein the added cloride source in step iii) is HCl.

4. A process according to claim 1, wherein the chiral ligand of the ruthenium catalyst complex has the formula (I) 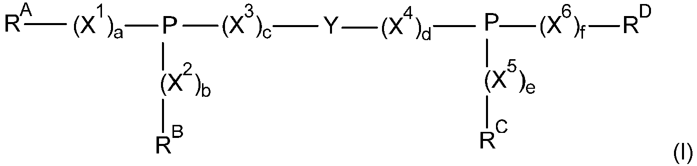 wherein
R^{A}, R^{B}, R^{C} and R^{D} are independently from each other selected from the group consisting of alkyl having 1 to 30 carbon atoms, cycloalkyl having 3 to 12 carbon ring members, heterocycloalkyl having 3 to 12 ring atoms, aryl, such as C₆-C₁₄-aryl, and hetaryl having 5 to 14 ring atoms,
wherein the alkyl radicals may be unsubstituted or carry 1, 2, 3, 4 or 5 substituents selected from cycloalkyl having 5 to 8 carbon ring members, heterocycloalkyl having 3 to 12 ring atoms, aryl, such as C₆-C₁₄-aryl, hetaryl having 5 to 14 ring atoms, alkoxy having 1 to 4 carbon atoms, cycloalkoxy having 5 to 8 carbon ring members, heterocycloalkoxy having 3 to 12 ring atoms, aryloxy, such as C₆-C₁₄-aryloxy, hetaryloxy having 5 to 14 ring atoms, hydroxy, mercapto, polyalkylene oxide, polyalkyleneimine, carboxyl, SO₃H, sulfonate, NE¹E², NE¹E²E³⁺X⁻, halogen, nitro, formyl, acyl and cyano, wherein E¹, E² and E³ are the same or different and are selected from hydrogen, alkyl, such as C₁-C₂₀-alkyl, cycloalkyl having 3 to 12 carbon ring members, and aryl, such as C₆-C₁₄-aryl, and X⁻ is an anion equivalent,
and wherein the radicals cycloalkyl, heterocycloalkyl, aryl and hetaryl in R^{A}, R^{B}, R^{C} and R^{D} may be unsubstituted or carry 1, 2, 3, 4 or 5 substituents selected from alkyl having 1 to 4 carbon atoms, cycloalkyl having 5 to 8 carbon ring members, heterocycloalkyl having 3 to 12 ring atoms, aryl, such as C₆-C₁₄-aryl, hetaryl having 5 to 14 ring atoms, alkoxy having 1 to 10 carbon atoms, cycloalkoxy having 5 to 8 carbon ring members, heterocycloalkoxy having 3 to 12 ring atoms, aryloxy, such as C₆-C₁₄-aryloxy, hetaryloxy having 5 to 14 ring atoms, hydroxy, mercapto, polyalkylene oxide, polyalkyleneimine, carboxyl, SO₃H, sulfonate, NE¹E², NE¹E²E³⁺X⁻, halogen, nitro, formyl, acyl and cyano, or
R^{A} and R^{B} and/or R^{C} and R^{D} together with the P atom and, if present, the groups X¹, X², X⁵ and X⁶ to which they are bound, are a 5- to 8-membered heterocycle, which is optionally fused with one, two or three groups selected from cycloalkyl having 5 to 8 carbon ring members, heterocycloalkyl having 3 to 12 ring atoms, aryl, such as C₆-C₁₄-aryl, hetaryl having 5 to 14 ring atoms, wherein the heterocycle and, if present, the fused-on groups independently from each other may each carry 1, 2, 3 or 4 substituents selected from alkyl having 1 to 10 carbon atoms, cycloalkyl having 5 to 8 carbon ring members, heterocycloalkyl having 3 to 12 ring atoms, aryl, such as C₆-C₁₄-aryl, hetaryl having 5 to 14 ring atoms, hydroxy, mercapto, polyalkylene oxide, polyalkyleneimine, alkoxy, halogen, carboxyl, SO₃H, sulfonate, NE⁴E⁵, NE⁴E⁵E⁶⁺X⁻, nitro, alkoxycarbonyl, such as C₁-C₂₀-alkoxycarbonyl, formyl, acyl and cyano, wherein E⁴, E⁵ and E⁶ are the same or different and are selected from hydrogen, alkyl, such as C₁-C₂₀-alkyl, cycloalkyl having 3 to 12 carbon ring members and aryl, such as C₆-C₁₄-aryl, and X⁻ is an anion equivalent,
X¹, X², X³, X⁴, X⁵, X⁶, X⁷, X⁸ and X⁹ are independently from each other O, S, CR^{x}R^{y}, SiR^{x}R^{y} or NR^{z}, wherein R^{x}, R^{y} and R^{z} are independently from each other hydrogen, alkyl having 1 to 4 carbon atoms, cycloalkyl having in particular 5 to 8 carbon ring members, heterocycloalkyl having 3 to 12 ring atoms, aryl, such as C₆-C₁₄-aryl, or hetaryl having 5 to 14 ring atoms,
Y is a divalent bridging group, which contains carbon atoms,
a, b, c, d, e and f are independently from each other 0 or 1,
provided that formula (I) has at least one chiral group, e.g. because at least one of the P atoms is asymmetric, and/or the ligand of formula (I) has axial chirality.

5. A process according to claim 4, wherein the divalent bridging group Y in formula (I) is selected from groups of the formulae (V) or (VI): 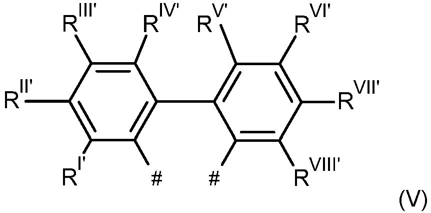 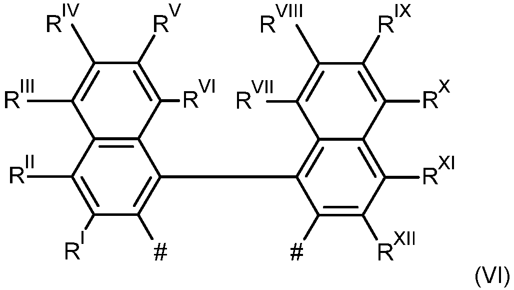 wherein
# denotes the binding sites to the remainder of the molecule,
R^{I}, R^{I'}, R^{II}, R^{II'}, R^{III}, R^{III'}, R^{IV}, R^{IV'}, R^{V}, R^{V'}, R^{VI}, R^{VI'}, R^{VII}, R^{VII'}, R^{VIII}, R^{VIII'} R^{IX}, R^{X}, R^{XI} and R^{XII} are each, independently from each other, hydrogen, alkyl having 1 to 20 carbon atoms, cycloalkyl having 3 to 12 carbon ring members, heterocycloalkyl having 3 to 12 ring atoms, C₆-C₁₄-aryl, hetaryl having 5 to 14 ring atoms, hydroxy, thiol, polyalkylene oxide, polyalkylenimine, alkoxy, halogen, SO₃H, sulfonate, NE¹E², alkylene-NE¹E², nitro, alkoxycarbonyl, carboxyl, acyl or cyano, wherein E¹ and E² are selected from the group consisting of hydrogen, C₁-C₂₀-alkyl, cycloalkyl having 3 to 12 carbon ring members and aryl,
wherein two adjacent radicals R^{I'}, R^{II'}, R^{III'}, R^{IV'}, R^{V'}, R^{VI'}, R^{VII'}, R^{VIII'} together with the carbon atoms of the benzene ring to which they are bound may also be a condensed ring system with 1, 2 or 3 further rings, wherein the ring atoms are selected from carbon, oxygen and sulfur, and wherein each of the rings may carry 1, 2 or 3 substituents selected from halogen, C₁-C₄-alkyl and C₁-C₄-alkoxy,
wherein two radicals R^{IV'} and R^{V'} together with the carbon atoms of the two benzene rings to which they are bound may also be a condensed ring system, wherein the ring atoms are selected from carbon, oxygen and sulfur, and wherein each of the rings may carry 1, 2 or 3 substituents selected from halogen C₁-C₄-alkyl and C₁-C₄-alkoxy.

6. A process according to any one of the preceding claims, wherein the ruthenium catalyst complex is present in such an amount, such that the amount of ruthenium is in the range from 0.001 mol% to 50 mol%, calculated as elemental ruthenium and based on the amount of L-Sorbose subjected to the hydrogenation.

7. A process according to any one of the preceding claims, wherein the chiral ligand is present in an amount of at least 0.5 mol per 1 mol of the ruthenium present.

8. A process according to any one of the preceding claims, wherein the reaction is carried out in the presence of a solvent selected from aliphatic hydrocarbons, aromatic hydrocarbons, amides, ureas, nitriles, sulfoxides, sulfones, alcohols, esters, carbonates, ethers, water and mixtures thereof.

9. The process according to any one of the preceding claims, wherein the ratio of D-Sorbitol to L-lditol is in the range of 1:7 to 1:1.5, preferably in the range of 1:6.5 to 1:1.9.

10. Use of a transition metal complex as defined in anyone of claims 4 to 5 as hydrogenation catalyst for the hydrogenation of compositions comprising L-Sorbose or mixtures thereof, **characterised in that**
(i) the hydrogenation is performed with hydrogen in homogeneous solution
(ii) the reaction products produced in step i) are separated from the ruthenium catalyst complex, and
(iii) the separated ruthenium catalyst complex of step ii) is reactivated by adding a chloride source and reused in step i).

## Patentansprüche

1. Verfahren zur Herstellung von L-Iditol, das mindestens die folgenden Verfahrensschritte umfasst:
i) eine L-Sorbose umfassende Zusammensetzung wird in Gegenwart eines hydrophoben stereoselektiven Rutheniumkatalysatorkomplexes in homogener Lösung mit Wasserstoff hydriert, wobei der Rutheniumkatalysatorkomplex mindestens einen chiralen Liganden mit mindestens zwei Phosphoratomen, die mit dem Ruthenium koordinieren können, umfasst, was eine Zusammensetzung ergibt, die L-Iditol als Hauptprodukt umfasst;
ii) Abtrennen der in Schritt i) hergestellten Reaktionsprodukte von dem Rutheniumkatalysatorkomplex;
iii) Reaktivieren des abgetrennten Rutheniumkatalysatorkomplexes aus Schritt ii) durch Zugeben einer Chloridquelle und Wiederverwenden des reaktivierten Rutheniumkatalysatorkomplexes in Schritt i).

2. Verfahren nach Anspruch 1, wobei die Abtrennung der Reaktionsprodukte in Schritt ii) als Extraktion mit Wasser durchgeführt wird.

3. Verfahren nach Anspruch 1, wobei es sich bei der zugegebenen Chloridquelle in Schritt iii) um HCl handelt.

4. Verfahren nach Anspruch 1, wobei der chirale Ligand des Rutheniumkatalysatorkomplexes die Formel (I) aufweist: 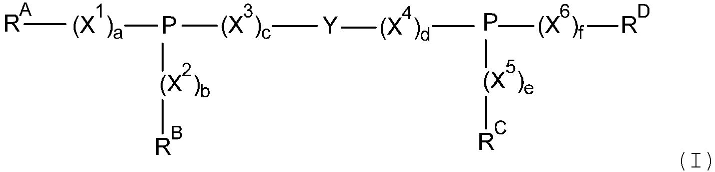 wobei
R^{A}, R^{B}, R^{C} und R^{D} unabhängig voneinander aus der Gruppe bestehend aus Alkyl mit 1 bis 30 Kohlenstoffatomen, Cycloalkyl mit 3 bis 12 Kohlenstoffringgliedern, Heterocycloalkyl mit 3 bis 12 Ringatomen, Aryl, wie C₆-C₁₄-Aryl, und Hetaryl mit 5 bis 14 Ringatomen ausgewählt sind,
wobei die Alkylreste unsubstituiert sein können oder 1, 2, 3, 4 oder 5 Substituenten tragen können, die aus Cycloalkyl mit 5 bis 8 Kohlenstoffringgliedern, Heterocycloalkyl mit 3 bis 12 Ringatomen, Aryl, wie C₆-C₁₄-Aryl, Hetaryl mit 5 bis 14 Ringatomen, Alkoxy mit 1 bis 4 Kohlenstoffatomen, Cycloalkoxy mit 5 bis 8 Kohlenstoffringgliedern, Heterocycloalkoxy mit 3 bis 12 Ringatomen, Aryloxy, wie C₆-C₁₄-Aryloxy, Hetaryloxy mit 5 bis 14 Ringatomen, Hydroxy, Mercapto, Polyalkylenoxid, Polyalkylenimin, Carboxyl, SO₃H, Sulfonat, NE¹E², NE¹E²E³⁺X⁻, Halogen, Nitro, Formyl, Acyl und Cyano ausgewählt sind, wobei E¹, E² und E³ gleich oder verschieden sind und aus Wasserstoff, Alkyl, wie C₁-C₂₀-Alkyl, Cycloalkyl mit 3 bis 12 Kohlenstoffringgliedern und Aryl, wie C₆-C₁₄-Aryl, ausgewählt sind, und X⁻ für ein Anionenäquivalent steht,
und wobei die Cycloalkyl-, Heterocycloalkyl-, Aryl- und Hetarylreste in R^{A}, R^{B}, R^{C} und R^{D} unsubstituiert sein können oder 1, 2, 3, 4 oder 5 Substituenten tragen können, die aus Alkyl mit 1 bis 4 Kohlenstoffatomen, Cycloalkyl mit 5 bis 8 Kohlenstoffringgliedern, Heterocycloalkyl mit 3 bis 12 Ringatomen, Aryl, wie C₆-C₁₄-Aryl, Hetaryl mit 5 bis 14 Ringatomen, Alkoxy mit 1 bis 10 Kohlenstoffatomen, Cycloalkoxy mit 5 bis 8 Kohlenstoffringgliedern, Heterocycloalkoxy mit 3 bis 12 Ringatomen, Aryloxy, wie C₆-C₁₄-Aryloxy, Hetaryloxy mit 5 bis 14 Ringatomen, Hydroxy, Mercapto, Polyalkylenoxid, Polyalkylenimin, Carboxyl, SO₃H, Sulfonat, NE¹E², NE¹E²E³⁺X⁻, Halogen, Nitro, Formyl, Acyl und Cyano ausgewählt sind, oder
R^{A} und R^{B} und/oder R^{C} und R^{D} zusammen mit dem P-Atom und, sofern vorhanden, den Gruppen X¹, X², X⁵ und X⁶, an die sie gebunden sind, für einen 5- bis 8-gliedrigen Heterocyclus stehen, der gegebenenfalls mit einer, zwei oder drei Gruppen, die aus Cycloalkyl mit 5 bis 8 Kohlenstoffringgliedern, Heterocycloalkyl mit 3 bis 12 Ringatomen, Aryl, wie C₆-C₁₄-Aryl, Hetaryl mit 5 bis 14 Ringatomen ausgewählt sind, anelliert ist, wobei der Heterocyclus und, sofern vorhanden, die anellierten Gruppen unabhängig voneinander jeweils 1, 2, 3 oder 4 Substituenten tragen können, die aus Alkyl mit 1 bis 10 Kohlenstoffatomen, Cycloalkyl mit 5 bis 8 Kohlenstoffringgliedern, Heterocycloalkyl mit 3 bis 12 Ringatomen, Aryl, wie C₆-C₁₄-Aryl, Hetaryl mit 5 bis 14 Ringatomen, Hydroxy, Mercapto, Polyalkylenoxid, Polyalkylenimin, Alkoxy, Halogen, Carboxyl, SO₃H, Sulfonat, NE⁴E⁵, NE⁴E⁵E⁶⁺X⁻, Nitro, Alkoxycarbonyl, wie C₁-C₂₀-Alkoxycarbonyl, Formyl, Acyl und Cyano ausgewählt sind, wobei E⁴, E⁵ und E⁶ gleich oder verschieden sind und aus Wasserstoff, Alkyl, wie C₁-C₂₀-Alkyl, Cycloalkyl mit 3 bis 12 Kohlenstoffringgliedern und Aryl, wie C₆-C₁₄-Aryl, ausgewählt sind und X⁻ für ein Anionäquivalent steht,
X¹, X², X³, X⁴, X⁵, X⁶, X⁷, X⁸ und X⁹ unabhängig voneinander für O, S, CR^{x}R^{y}, SiR^{x}R^{y} oder NR^{z} stehen, wobei R^{x}, R^{y} und R^{z} unabhängig voneinander für Wasserstoff, Alkyl mit 1 bis 4 Kohlenstoffatomen, Cycloalkyl mit insbesondere 5 bis 8 Kohlenstoffringgliedern, Heterocycloalkyl mit 3 bis 12 Ringatomen, Aryl, wie C₆-C₁₄-Aryl, oder Hetaryl mit 5 bis 14 Ringatomen stehen,
Y für eine zweiwertige Brückengruppe, die Kohlenstoffatome enthält, steht,
a, b, c, d, e und f unabhängig voneinander für 0 oder 1 stehen,
mit der Maßgabe, dass Formel (I) mindestens eine chirale Gruppe aufweist, z. B. weil mindestens eines der P-Atome asymmetrisch ist und/oder der Ligand der Formel (I) axiale Chiralität aufweist.

5. Verfahren nach Anspruch 4, wobei die zweiwertige Brückengruppe Y in Formel (I) aus Gruppen der Formeln (V) oder (VI) ausgewählt ist: 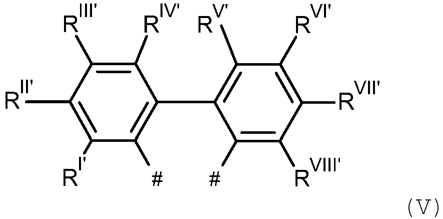 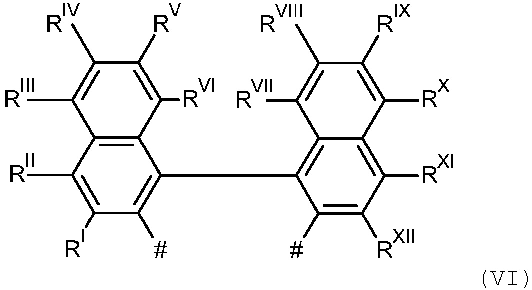 wobei
# die Anbindungsstellen an den Rest des Moleküls anzeigt, R^{I}, R^{I'}, R^{II}, R^{II'}, R^{III}, R^{III'}, R^{IV}, R^{IV'}, R^{V}, R^{V'}, R^{VI}, R^{VI'}, R^{VII}, R^{VII'}, R^{VIII}, R^{VIII'}, R^{IX}, R^{X}, R^{XI} und R^{XII} jeweils unabhängig voneinander für Wasserstoff, Alkyl mit 1 bis 20 Kohlenstoffatomen, Cycloalkyl mit 3 bis 12 Kohlenstoffringgliedern, Heterocycloalkyl mit 3 bis 12 Ringatomen, C₆-C₁₄-Aryl, Hetaryl mit 5 bis 14 Ringatomen, Hydroxy, Thiol, Polyalkylenoxid, Polyalkylenimin, Alkoxy, Halogen, SO₃H, Sulfonat, NE¹E², Alkylen-NE¹E², Nitro, Alkoxycarbonyl, Carboxyl, Acyl oder Cyano stehen, wobei E¹ und E² aus der Gruppe bestehend aus Wasserstoff, C₁-C₂₀-Alkyl, Cycloalkyl mit 3 bis 12 Kohlenstoffringgliedern und Aryl ausgewählt sind,
wobei zwei benachbarte Reste R^{I'}, R^{II'}, R^{III'}, R^{IV'}, R^{V'}, R^{VI'}, R^{VII'}, R^{VIII'} zusammen mit den Kohlenstoffatomen des Benzolrings, an den sie gebunden sind, auch für ein kondensiertes Ringsystem mit 1, 2 oder 3 weiteren Ringen stehen können, wobei die Ringatome aus Kohlenstoff, Sauerstoff und Schwefel ausgewählt sind und wobei jeder der Ringe 1, 2 oder 3 Substituenten tragen kann, die aus Halogen, C₁-C₄-Alkyl und C₁-C₄-Alkoxy ausgewählt sind,
wobei zwei Reste R^{IV'} and R^{V'} zusammen mit den Kohlenstoffatomen der zwei Benzolringe, an die sie gebunden sind, auch für ein kondensiertes Ringsystem stehen können, wobei die Ringatome aus Kohlenstoff, Sauerstoff und Schwefel ausgewählt sind und wobei jeder der Ringe 1, 2 oder 3 Substituenten tragen kann, die aus Halogen, C₁-C₄-Alkyl und C₁-C₄-Alkoxy ausgewählt sind.

6. Verfahren nach einem der vorhergehenden Ansprüche, wobei der Rutheniumkatalysatorkomplex in einer solchen Menge vorliegt, dass die Menge an Ruthenium im Bereich von 0,001 Mol-% bis 50 Mol-%, berechnet als elementares Ruthenium und bezogen auf die der Hydrierung unterworfene Menge an L-Sorbose, vorliegt.

7. Verfahren nach einem der vorhergehenden Ansprüche, wobei der chirale Ligand in einer Menge von mindestens 0,5 mol pro 1 mol des vorhandenen Rutheniums vorliegt.

8. Verfahren nach einem der vorhergehenden Ansprüche, wobei die Umsetzung in Gegenwart eines aus aliphatischen Kohlenwasserstoffen, aromatischen Kohlenwasserstoffen, Amiden, Harnstoffen, Nitrilen, Sulfoxiden, Sulfonen, Alkoholen, Estern, Carbonaten, Ethern, Wasser und Mischungen davon ausgewählten Lösungsmittels durchgeführt wird.

9. Verfahren nach einem der vorhergehenden Ansprüche, wobei das Verhältnis von D-Sorbitol zu L-Iditol im Bereich von 1:7 bis 1:1,5, vorzugsweise im Bereich von 1:6,5 bis 1:1,9, liegt.

10. Verwendung eines wie in einem der Ansprüche 4 bis 5 definierten Übergangsmetallkomplexes als Hydrierkatalysator für die Hydrierung von Zusammensetzungen, die L-Sorbose oder Mischungen davon umfassen, **dadurch gekennzeichnet, dass**
(i) die Hydrierung mit Wasserstoff in homogener Lösung durchgeführt wird,
(ii) die in Schritt i) hergestellten Reaktionsprodukte von dem Rutheniumkatalysatorkomplex abgetrennt werden und
(iii) der abgetrennte Rutheniumkatalysatorkomplex aus Schritt ii) durch Zugeben einer Chloridquelle reaktiviert und in Schritt i) wiederverwendet wird.

## Revendications

1. Procédé de préparation de L-iditol comprenant au moins les étapes de procédé suivantes :
i) une composition comprenant du L-sorbose est soumise à une hydrogénation à l'aide d'hydrogène en présence d'un complexe catalyseur au ruthénium stéréosélectif hydrophobe dans une solution homogène, le complexe catalyseur au ruthénium comprenant au moins un ligand chiral contenant au moins deux atomes de phosphore, qui sont capables de se coordonner au ruthénium pour obtenir une composition contenant du L-iditol en tant que produit principal ;
ii) séparation des produits de réaction obtenus à l'étape i) du complexe catalyseur au ruthénium ;
iii) réactivation du complexe catalyseur au ruthénium séparé de l'étape ii) par ajout d'une source de chlorure et réutilisation du complexe catalyseur au ruthénium réactivé à l'étape i).

2. Procédé selon la revendication 1, dans lequel la séparation des produits de réaction à l'étape ii) est réalisée sous la forme d'une extraction avec de l'eau.

3. Procédé selon la revendication 1, dans lequel la source de chlorure ajoutée à l'étape iii) est HCl.

4. Procédé selon la revendication 1, dans lequel le ligand chiral du complexe catalyseur au ruthénium a la formule (I) 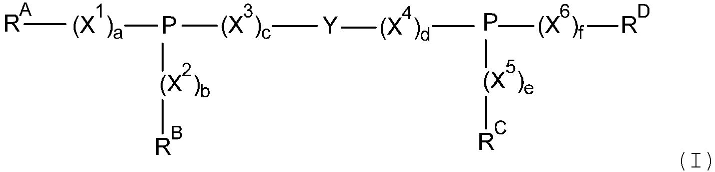 dans lesquelles
R^{A}, R^{B}, R^{C} et R^{D} sont, indépendamment les uns des autres, choisis dans le groupe constitué par alkyle ayant 1 à 30 atomes de carbone, cycloalkyle ayant 3 à 12 chaînons cycliques de carbone, hétérocycloalkyle ayant 3 à 12 atomes cycliques, aryle, tel qu'aryle en C₆-C₁₄, et hétéroaryle ayant 5 à 14 atomes cycliques,
dans laquelle les radicaux alkyle peuvent être non substitués ou comporter 1, 2, 3, 4 ou 5 substituants choisis parmi cycloalkyle ayant 5 à 8 chaînons cycliques de carbone, hétérocycloalkyle ayant 3 à 12 atomes cycliques, aryle, tel qu'aryle en C₆-C₁₄, hétéroaryle ayant 5 à 14 atomes cycliques, alcoxy ayant 1 à 4 atomes de carbone, cycloalcoxy ayant 5 à 8 chaînons cycliques de carbone, hétérocycloalcoxy ayant 3 à 12 atomes cycliques, aryloxy, tel qu'aryloxy en C₆-C₁₄, hétéroaryloxy ayant 5 à 14 atomes cycliques, hydroxy, mercapto, poly(oxyde d'alkylène), polyalkylèneimine, carboxyle, SO₃H, sulfonate, NE¹E², NE¹E²E³⁺X⁻, halogène, nitro, formyle, acyle et cyano, dans laquelle E¹, E² et E³ sont identiques ou différents et sont choisis parmi hydrogène, alkyle, tel qu'alkyle en C₁-C₂₀, cycloalkyle ayant 3 à 12 chaînons cycliques de carbone, et aryle, tel qu'aryle en C₆-C₁₄, et X⁻ est un équivalent anionique,
et dans laquelle les radicaux cycloalkyle, hétérocycloalkyle, aryle et hétéroaryle dans R^{A}, R^{B}, R^{C} et R^{D} peuvent être non substitués ou comporter 1, 2, 3, 4 ou 5 substituants choisis parmi alkyle ayant 1 à 4 atomes de carbone, cycloalkyle ayant 5 à 8 chaînons cycliques de carbone, hétérocycloalkyle ayant 3 à 12 atomes cycliques, aryle, tel qu'aryle en C₆-C₁₄, hétéroaryle ayant 5 à 14 atomes cycliques, alcoxy ayant 1 à 10 atomes de carbone, cycloalcoxy ayant 5 à 8 chaînons cycliques de carbone, hétérocycloalcoxy ayant 3 à 12 atomes cycliques, aryloxy, tel qu'aryloxy en C₆-C₁₄, hétéroaryloxy ayant 5 à 14 atomes cycliques, hydroxy, mercapto, poly(oxyde d'alkylène), polyalkylèneimine, carboxyle, SO₃H, sulfonate, NE¹E², NE¹E²E³⁺X⁻, halogène, nitro, formyle, acyle et cyano, ou
R^{A} et R^{B} et/ou R^{C} et R^{D}, conjointement avec l'atome P et, s'ils sont présents, les groupes X¹, X², X⁵ et X⁶ auxquels ils sont liés, forment un hétérocycle de 5 à 8 chaînons, qui est éventuellement condensé avec un, deux ou trois groupes choisis parmi cycloalkyle ayant 5 à 8 chaînons cycliques de carbone, hétérocycloalkyle ayant 3 à 12 atomes cycliques, aryle, tel qu'aryle en C₆-C₁₄, hétéroaryle ayant 5 à 14 atomes cycliques, dans laquelle l'hétérocycle et, s'ils sont présents, les groupes condensés pouvant chacun comporter indépendamment 1, 2, 3 ou 4 substituants choisis parmi alkyle ayant 1 à 10 atomes de carbone, cycloalkyle ayant 5 à 8 chaînons cycliques de carbone, hétérocycloalkyle ayant 3 à 12 atomes cycliques, aryle, tel qu'aryle en C₆-C₁₄, hétéroaryle ayant 5 à 14 atomes cycliques, hydroxy, mercapto, poly(oxyde d'alkylène), polyalkylèneimine, alcoxy, halogène, carboxyle, SO₃H, sulfonate, NE⁴E⁵, NE⁴E⁵E⁶⁺X⁻, nitro, alcoxycarbonyle, tel qu'alcoxycarbonyle en C₁-C₂₀, formyle, acyle et cyano, dans laquelle E⁴, E⁵ et E⁶ sont identiques ou différents et sont choisis parmi hydrogène, alkyle, tel qu'alkyle en C₁-C₂₀, cycloalkyle ayant 3 à 12 chaînons cycliques de carbone et aryle, tel qu'aryle en C₆-C₁₄, et X⁻ est un équivalent anionique,
X¹, X², X³, X⁴, X⁵, X⁶, X⁷, X⁸ et X⁹ sont, indépendamment les uns des autres, O, S, CR^{x}R^{y}, SiR^{x}R^{y} ou NR^{z}, dans laquelle R^{x}, R^{y} et R^{z} sont, indépendamment les uns des autres, hydrogène, alkyle ayant 1 à 4 atomes de carbone, cycloalkyle ayant notamment 5 à 8 chaînons cycliques de carbone, hétérocycloalkyle ayant 3 à 12 atomes cycliques, aryle, tel qu'aryle en C₆-C₁₄, ou hétéroaryle ayant 5 à 14 atomes cycliques,
Y est un groupe pontant divalent, qui contient des atomes de carbone,
a, b, c, d, e et f sont, indépendamment les uns des autres, 0 ou 1,
à condition que la formule (I) comporte au moins un groupe chiral, par exemple parce qu'au moins un des atomes de phosphore est asymétrique, et/ou que le ligand de formule (I) présente une chiralité axiale.

5. Procédé selon la revendication 4, dans lequel le groupe pontant divalent Y dans la formule (I) est choisi parmi des groupes de formules (V) ou (VI) : 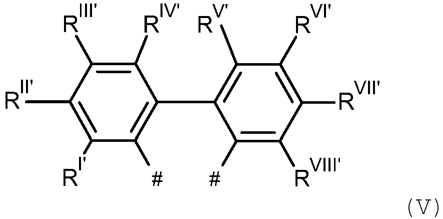 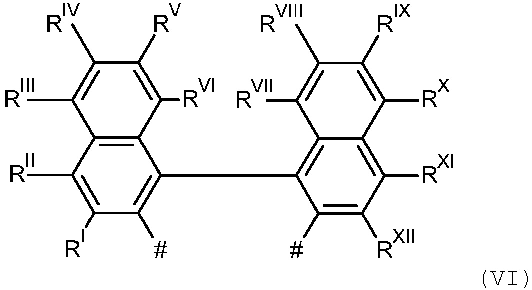 dans lesquelles
# désigne les sites de liaison au reste de la molécule, R^{I}, R^{I'}, R^{II}, R^{II'}, R^{III}, R^{III'}, R^{IV}, R^{IV'}, R^{V}, R^{V'}, R^{VI}, R^{VI'}, R^{VII}, R^{VII'}, R^{VIII}, R^{VIII'} R^{IX}, R^{X}, R^{XI} et R^{XII} sont chacun, indépendamment les uns des autres, hydrogène, alkyle ayant 1 à 20 atomes de carbone, cycloalkyle ayant 3 à 12 chaînons cycliques de carbone, hétérocycloalkyle ayant 3 à 12 atomes cycliques, aryle en C₆-C₁₄, hétéroaryle ayant 5 à 14 atomes cycliques, hydroxy, thiol, poly(oxyde d'alkylène), polyalkylèneimine, alcoxy, halogène, SO₃H, sulfonate, NE¹E², alkylène-NE¹E², nitro, alcoxycarbonyle, carboxyle, acyle ou cyano, dans laquelle E¹ et E² sont choisis dans le groupe constitué par hydrogène, alkyle en C₁-C₂₀, cycloalkyle ayant 3 à 12 chaînons cycliques de carbone et aryle,
dans laquelle deux radicaux adjacents R^{I'}, R^{II'}, R^{III'}, R^{IV'}, R^{V'}, R^{VI'}, R^{VIII'}, conjointement avec les atomes de carbone du cycle benzénique auxquels ils sont liés peuvent également être un système cyclique condensé avec 1, 2 ou 3 cycles supplémentaires, les atomes cycliques étant choisis parmi carbone, oxygène et soufre, et chacun des cycles pouvant comporter 1, 2 ou 3 substituants choisis parmi halogène, alkyle en C₁-C₄ et alcoxy en C₁-C₄
deux radicaux R^{IV'} et ^{RV'}, conjointement avec les atomes de carbone des deux cycles benzéniques auxquels ils sont liés, peuvent également être un système cyclique condensé, les atomes cycliques étant choisis parmi carbone, oxygène et soufre, et chacun des cycles pouvant comporter 1, 2 ou 3 substituants choisis parmi halogène alkyle en C₁-C₄ et alcoxy en C₁-C₄.

6. Procédé selon l'une quelconque des revendications précédentes, dans lequel le complexe de catalyseur de métal de transition est présent en une quantité telle que la quantité de ruthénium est dans la plage de 0,001 % en moles à 50 % en moles, calculée en ruthénium élémentaire et par rapport à la quantité de L-sorbose soumise à l'hydrogénation.

7. Procédé selon l'une quelconque des revendications précédentes, dans lequel le ligand chiral est présent en une quantité d'au moins 0,5 mole pour 1 mole du ruthénium présent.

8. Procédé selon l'une quelconque des revendications précédentes, dans lequel la réaction est conduite en présence d'un solvant choisi parmi des hydrocarbures aliphatiques, des hydrocarbures aromatiques, des amides, des urées, des nitriles, des sulfoxydes, des sulfones, des alcools, des esters, des carbonates, des éthers, l'eau et des mélanges correspondants.

9. Procédé selon l'une quelconque des revendications précédentes, dans lequel le rapport du D-sorbitol au L-iditol est dans la plage de 1:7 à 1:1,5, de préférence dans la plage de 1:6,5 à 1:1,9.

10. Utilisation d'un complexe de métaux de transition tel que défini dans l'une quelconque des revendications 4 à 5 en tant que catalyseur d'hydrogénation pour l'hydrogénation de compositions comprenant du L-sorbose ou des mélanges correspondants, **caractérisée en ce que**
(i) l'hydrogénation est réalisée avec de l'hydrogène en solution homogène
(ii) les produits de réaction produits à l'étape i) sont séparés du complexe catalyseur au ruthénium, et
(iii) le complexe catalyseur au ruthénium séparé de l'étape ii) est réactivé par ajout d'une source de chlorure et réutilisé à l'étape i).
